(19) Europäisches Patentamt
European Patent Office
Office européen des brevets

(11) Veröffentlichungsnummer: **0 578 026 A1**

# (12) EUROPÄISCHE PATENTANMELDUNG

(21) Anmeldenummer: **93109694.5**

(22) Anmeldetag: **17.06.93**

(51) Int. Cl.[5]: **C07H 15/10**, C07H 13/04, C07H 15/04, A61K 31/70

(30) Priorität: **30.06.92 DE 4221444**

(43) Veröffentlichungstag der Anmeldung: **12.01.94 Patentblatt 94/02**

(84) Benannte Vertragsstaaten: **AT BE CH DE DK ES FR GB GR IE IT LI LU NL PT SE**

(71) Anmelder: **Merck Patent GmbH**
**Postfach,**
**Frankfurter Strasse 250**
**D-64271 Darmstadt(DE)**

(72) Erfinder: **Schmidt, Richard, Prof. Dr.**
**Grossherzog-Friedrich-Strasse 11**
**D-7750 Konstanz 16(DE)**
Erfinder: **Toepfer, Alexander**
**Kirschgartenstr. 12**
**D-65719 Hofheim (a.Taunus)(DE)**
Erfinder: **Kinzy, Willy, Dr.**
**Eggenweg 13**
**D-7850 Lörrach(DE)**
Erfinder: **Hemberger, Jürgen, Dr.**
**Konradstrasse 7a**
**D-8750 Aschaffenburg(DE)**

(54) **Polymere Lewis X-Saccharide und Verfahren zu ihrer Herstellung.**

(57) Die Erfindung betrifft ein Verfahren zur Herstellung polymerer Lewis X-Saccharide der Formel

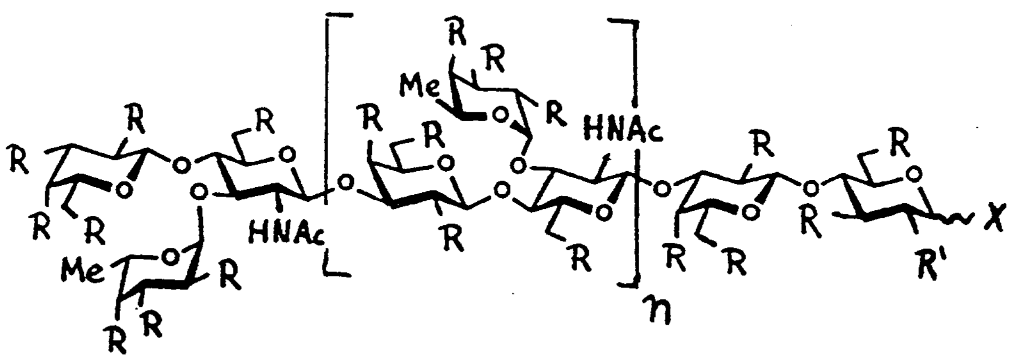

worin

| | |
|---|---|
| X | $OC(=NH)CCl_3$ (TCAI), Z oder S, |
| Z | $OCH_2CH(NHCOC_uH_{2u+1})CH(OH)CH=CHC_vH_{2v+1}$, |
| S | $(CH_2)_qCOOR''$, |
| R | OH, $R^{sg}$, |
| $R^{sg}$ | OAc, OBn oder OBz, |
| R' | OH, OAc oder OPiv, |
| R'' | $C_1$ bis $C_4$-Alkyl, |
| Ac | Acetyl, |
| Bn | Benzyl, |
| Bz | Benzoyl, |
| Piv | Pivaloyl, |

EP 0 578 026 A1

n eine ganze Zahl von 0 bis 7,
q eine ganze Zahl von 4 bis 12,
u eine ganze Zahl von 13 bis 23 und
v eine ganze Zahl von 11 bis 17

bedeuten,
ein Verfahren zur Herstellung entsprechender Zwischenprodukte,
sowie neue polare Lewis X-Saccharide, insbesondere tetrameres bis octameres Lewis X-Antigen.

Die polymeren Saccharide können in hohen Ausbeuten stereoselektiv synthetisiert werden.

Die Erfindung betrifft polymere Lewis X-Saccharide und ein neues effektives Verfahren zu ihrer Herstellung. Insbesondere betrifft die Erfindung di- bis octamere Lewis X-Derivate.

Die biologische Bedeutung von Glycokonjugaten, insbesondere Glycolipiden bzw. Glycosphingolipiden in ihrer Rolle als tumor-assoziierte Antigene auf dem Gebiet der Tumortherapie und Tumordiagnostik (z.B. Hakomori (1985), Cancer Res. 45, 2405) hat ein großes Interesse an chemischen Synthesen vor allem am Oligosaccharid-Anteil hervorgerufen. Die Oligosaccharid-Epitope können als Haptene fungieren und Antikörper initiieren, die als potentielle Kandidaten für die therapeutische oder diagnostische Behandlung von Carcinomen und deren Metastasen oder zur Stimulation der körpereigenen Abwehr gegen Tumorzellen eingesetzt werden können (z.B. Castronovo et al. (1989), J. Natl. Cancer Inst. 81 (3), 212).

Ein bekanntes tumor-assoziiertes Antigen ist das Lewis Antigen X ($Le^X$), dessen Oligosaccharid-Komponente sich aus Gal$\beta$(1→ 4)[Fuc$\alpha$→ 3)]GlcNAc zusammensetzt. Lewis Antigen X ist in monomerer, di- und trimerer Formbekannt (z.B. Holmes et al. (1987), J. Biol. Chem. 262, 11331 u. 15649).

Frühere Synthesen des $Le^X$ Antigens konzentrierten sich auf verschiedene Synthese-Strategien, Einsatz unteschiedlicher Synthese-Bausteine und Verwendung unterschiedlicher Schutzgruppen. So wurden beispielsweise 4-O-ungeschützte Glucosamin- Akzeptoren mit Galactosyl-Donoren umgesetzt und nach 3-O-Entschützung an einen Fucosylrest angekoppelt (z.B.: Nicolaou et al. (1990), J. Am. Chem. Soc. 112, 3693; Nicolaou et al. (1992), J. Am. Chem. Soc. 114, 3126; Sato et al. (1988), Tetrahedron Lett. 29, 5267; Sato et al. (1987), Carbohydr. Res. 167, 197; Hindsgaul et al. (1982), Carbohydr. Res. 109, 109; Lönn (1985), Carbohydr. Res. 139, 115; Classon et al. (1989), J. Carbohydr. Chem. 8, 543). Alternativ wurde auch die Kettenverlängerung entsprechender Lactosamin-Derivate mit anschließender Fucosylierung durchgeführt (z.B.: Nillson et al. (1987), Glcokonjugate J. 4, 219; Nillson et al. (1988), Carbohydr. Res. 183, 183), oder eine Strategie, basierend auf Azidolactose, entwickelt (z.B. Bommer et al. (1990), Liebigs Ann. Chem. 425). Weitere Verbesserungen ließen sich durch alpha-Fucosylierung mit Fucosyl-Donoren unter Verwendung der "Inversen Prozedur" (z.B. Schmidt et al. (1991), Tetrahedron Lett. 32, 3353) oder durch Herstellung der $\beta$-glycosidischen Bindung mittels 2-Azidoglycosyl-Donoren unter Verwendung des "Nitril Effekts" (z.B. Schmidt et al. (1990), Synlett 694; Vankar et al. (1991), Tetrahedron Lett. 47, 9985) erreichen. Mit dieser Methodik wird unter anderem eine hohe Stereoselektivität insbesondere beim Glycosylierungs-Schritt erzielt. Diese ist erforderlich, damit die vorhergesagte biologische Aktivität erhalten bleibt.

Trotz der genannten optimierenden Maßnahmen ist zu beobachten, daß die Ausbeuten bei der Synthese von den bekannten Tetra-, Hexa- und Octasacchariden mit zunehmender Kettenlänge in der Regel signifikant abnehmen. Demnach war zu erwarten, daß die Anwendung der bekannten Methodik auf die Synthese höherer polymerer Oligosaccharide, die für die genannten Anwendungen ebenfalls von großem Interesse sind, noch geringere Ausbeuten und somit eine unzureichende Wirtschaftlichkeit liefert.

Vorallem konnte nicht erwartet werden, daß tri-, tetra-, hexa- oder gar octamere Lewis X-Derivate, also Oligosaccharide, die sich aus elf, vierzehn, zwanzig oder sechsundzwanzig Monosaccharid-Bausteinen zusammensetzen, mit der bekannten Methodik in zufriedenstellenden Ausbeuten zugänglich sind.

Es bestand somit die Aufgabe, die für niedrigere $Le^X$-Bausteine bewährte Methodik in der Weise zu verändern und zu optimieren, daß auch über Octasaccharide hinausgehende Derivate in praktikabler Weise synthetisch zugänglich sind.

Es wurde nun gefunden, daß polymere Lewis X-Saccharide, durch beliebige Kettenverlängerung ausgehend von $Le^X$-Hexasacchariden unter Verwendung entsprechender Donor- und Akzeptorbausteine hergestellt werden können, wobei als Donor-Bausteine Trichloracetimidate (TCAI) und als Akzeptor-Bausteine Saccharide mit sterisch anspruchsvollen Schutzgruppen, insbesondere O-tert-Butyldimethylsilyl-Verbindungen (OTBS) von Vielfachen von $Le^X$ Trisaccharid-Elementen, gewünschtenfalls zuzüglich mit einem als Akzeptor fungierenden Di-Saccharid, eingesetzt werden.

Erfindungsgemäß zeichnen sich die genannten Akzeptor-Verbindungen durch Aryliden-, insbesondere 4,6-O-Benzyliden-Schutzgruppen vorallem an den endständigen Galactopyranose-Ringen aus, so daß ein leichter selektiver Zugang zu der jeweiligen 3-O-Position möglich ist, die für die Glycosylakzeptor-Eigenschaften verantwortlich ist.

Es wurde außerdem gefunden, daß die $Le^X$-Hexasaccharide sich über entsprechende Trisaccharide aus dem leicht zugänglichen tert-Butyldimethylsily-2-azido-4,6-O-benzyliden-glucopyranosid oder einem entsprechenden anderweitig silylierten und/oder arylidenierten 2-Azido-glucopyranosid effektiv herstellen lassen, welche ebenfalls das oben genannte Schutzgruppenmuster mit den entsprechenden Eigenschaften zur Bereitstellung von Donor- und Akkzeptorfunktionen aufweisen.

Man erhält somit polymere $Le^X$-Derivate mit Stereoselektivitätsraten von 90 bis 100 %, vorzugsweise von 95 bis 99 % und Ausbeuten zwischen 65 und 85 %, vorzugsweise zwischen 70 und 80 %, bezogen auf jeweils einen KettenverlängerungsZyclus. Überraschenderweise nehmen die Ausbeuten mit zunehmender Kettenlänge z.B. von den dimeren über die tetrameren bis zu den octameren Lewis X - Derivaten der

erfindungsgemäßen Verbindungen nicht ab. Selbst eine Kettenverlängerung darüber hinaus (n > 7, Formel I, IV) führt zu analogen Ergebnissen und lassen den Schluß zu, daß mit Hilfe des erfindungsgemäßen Verfahrens sogar hochpolymere Lewis X-Saccharide zur Verfügung gestellt werden können.

Gegenstand der Erfindung ist somit Verfahren zur Herstellung von polymeren Lewis X-Sacchariden der Formel I,

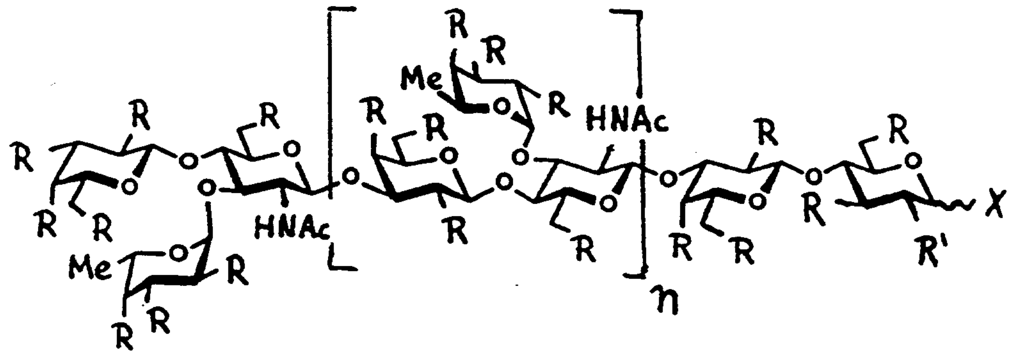

worin

| | |
|---|---|
| X | $OC(=NH)CCl_3$ (TCAI), Z oder S, |
| Z | $OCH_2CH(NHCOC_uH_{2u+1})CH(OH)CH=CHC_vH_{2v+1}$, |
| S | $(CH_2)_qCOOR''$, |
| R | OH, $R^{sg}$, |
| $R^{sg}$ | OAc, OBn oder OBz, |
| R' | OH, OAc oder OPiv, |
| R" | $C_1$ bis $C_4$-Alkyl, |
| Ac | Acetyl, Bn Benzyl, Bz Benzoyl, |
| Piv | Pivaloyl, |
| n | eine ganze Zahl von 0 bis 7, |
| q | eine ganze Zanl von 4 bis 12, |
| u | eine ganze Zahl von 13 bis 23 und |
| v | eine ganze Zahl von 11 bis 17 |

bedeuten, ausgehend von einem Hexasaccharid-Baustein, dadurch gekennzeichnet, daß folgende Schritte durchgeführt werden:

(a) Glycosidierung eines an der 1-O-Position aktivierten Donorsaccharid-Bausteins (II [TCAI/(x)]) ausgewählt aus einer der Verbindungen der Formel II,

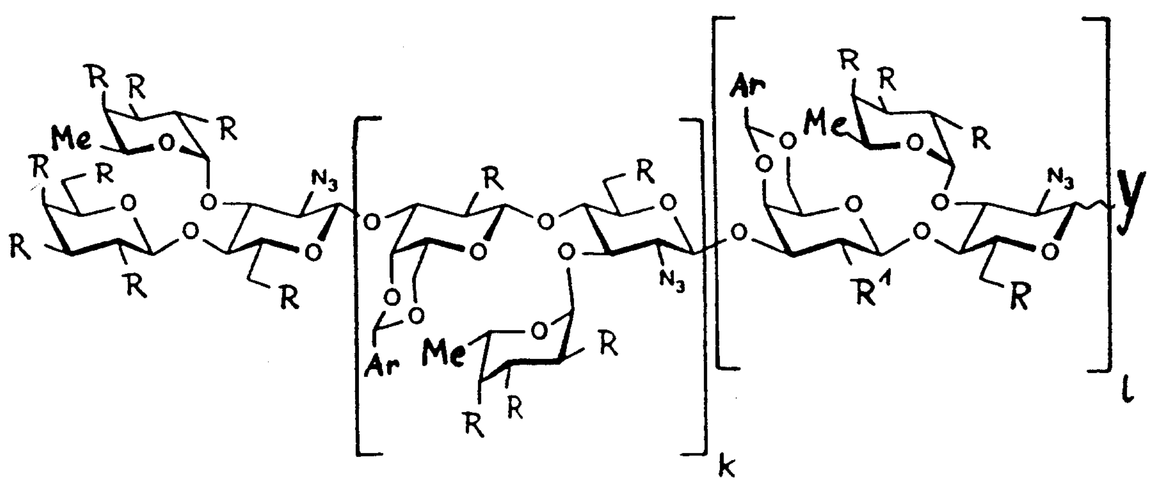

worin

| | |
|---|---|
| Y | $OC(=NH)CCl_3$ (TCAI) oder W, |
| W | O-tert-Butyldimethylsilyl (OTBS), O-Thexyldimethylsilyl (OTDS) oder O-tert-Butyldiphenylsiyl |

(OTDPS),
R wie angegeben,
$R^1$ R,
Ar Aryl,
Me Methyl,
k, l eine ganze Zahl von 0 bis 6
bedeuten,
wobei, wenn Y TCAI bedeutet, $R^1$ OAc, OBn oder OBz ist, und wenn Y W bedeutet, $R^1$ OH ist, mit einem Akzeptorsaccharid-Baustein der Formel V
( V [(y)] ),

V

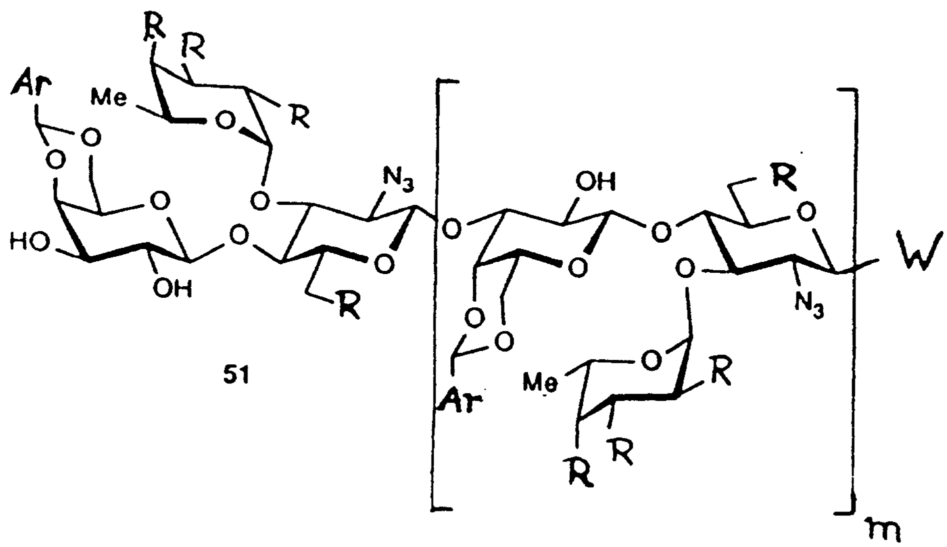

51

worin R, Ar, Me und W die angegebenen Bedeutungen haben und m eine ganze Zahl von 0 bis 5 ist, zu einem höherkettigen Saccharid der Formel II (II [W (x + y)], wobei x 6, 9, 12, 15, 18, 21 und y 3, 6, 9, 12, 15 und 18 bedeuten und die Anzahl der Monosaccharid-Elemente in dem betreffenden Polysaccharid wiedergeben, und x + y nicht größer als 24 sein dürfen;
(b) Umsetzung in das entsprechende höherkettige Donorsaccharid der Formel II ( II [TCAI/(x + y)] ) durch Substitution der freien OH-Gruppe (n) durch eine entsprechende Schuczgruppe und des W-Restes durch den TCAI-Rest;
(c) Glycosidierung des an der 1-O-Position aktivierten Donorsaccharids aus (b) mit einem als Akzeptor wirksamen Disaccharids der Formel III,

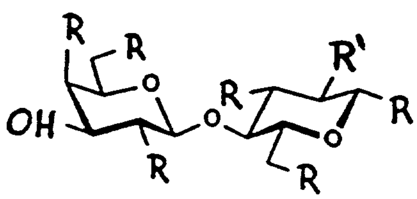

III

worin R und R' die angegebenen Bedeutungen haben, zu einem Saccharid der Formel IV [(x + y + 2)],

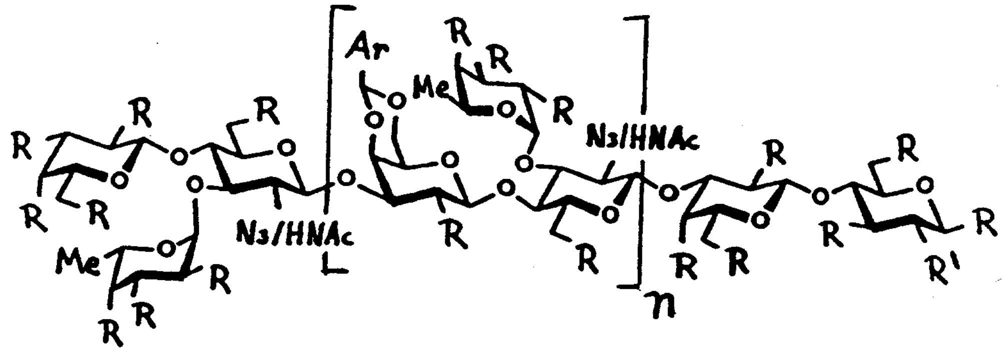

IV

worin R, R', Ar, Me und n die angegebenen Bedeutungen haben; und

(d) Reduktion der Azidogruppen zu Acetamidogruppen der Verbindung IV, Abspaltung der Aryliden- und Benzyl-Reste und Peracetylierung der Reste R und R', Substitution der glycosidischen Acetylgruppe durch OH und Umsetzung zum entsprechenden Trichloracetimidat (X = TCAI) der Formel I und gewünschtenfalls Modifizierung des Restes X zu Z oder S und Entfernung der Schutzgruppen (R, R' = OH).

Gegenstand der Erfindung ist ferner ein Verfahren zur Herstellung von Lewis X-Hexasacchariden der Formel II (II [TCAI/W/(x)]), k = 0, l = 1, x = 6), dadurch gekennzeichnet, daß man ein mit einer sterisch anspruchsvollen Silylschutzgruppe ausgestattetes Aryliden 2-Azido-glucopyranosid, insbesondere aber tert-Butyldimethylsilyl-2-azid-4,6-O-benzyliden-glucopyranosid mit Trichloracetimidaten entsprechender Monosaccharide zu einem Trisaccharid der Formel II (II [W/(x)], k = 0, l = 0, x = 3) umsetzt, aus diesem ein Donor-Trisaccharid der Formel II ( II [TCAI/(x)], x = 3) und ein Akzeptor-Trisaccharid der Formel V ( V [(y)], m = 0, y = 3) herstellt, welche miteinander zu dem entsprechenden Hexasaccharid umgesetzt werden.

Mit Hilfe des erfindungsgemäßen Verfahrens können auch neue polymere Lewis X-Saccharide chemisch synthetisiert werden, insbesondere das tetramere, hexamere und octamere Lewis X-Antigen.

Gegenstand der Erfindung sind somit auch polymere Lewis X-Saccharide der Formel I mit den oben angegebenen Bedeutungen, wobei jedoch n eine ganze Zahl von 2 bis 7 bedeutet mit der Maßgabe, daß, wenn n 2 bedeutet, X nicht Z mit u = 15 und v = 13 und R, R' nicht OH sein dürfen. Insbesondere sind dies:

tetrameres Lewis X-Antigen der Formel I (n = 3),
hexameres Lewis X-Antigen der Formel I (n = 5) und
octameres Lewis X-Antigen der Formel I (n = 7).

Weiterhin sind Gegenstand der Erfindung polymere Lewis X-Saccharide der Formel II mit den oben angegebenen Bedeutungen, wobei jedoch k 1 ist und l eine ganze Zahl von 1 bis 5 bedeutet, sowie polymere Lewis X-Saccharide der Formel IV mit den genannten Bedeutungen, wobei jedoch n eine ganze Zahl von 2 bis 7 bedeutet und letztlich polymere Lewis X-Saccharide der Formel V mit den genannten Bedeutungen, wobei jedoch m eine ganze Zahl von 1 bis 5 bedeutet.

Die Verbindungen der Formeln I, II, IV und V gemäß der Ansprüche 7 bis 11 eignen sich für den Einsatz in der Tumordiagnostik sowie zur Herstellung von Antikörpern für die Tumortherapie. Dabei kommen solche Tumoren in Frage, die die entsprechenden polymeren Lewis X-Antigene auf ihrer Oberfläche tragen. Beispiele für solche Tumoren können sein:
gastrointestinale Tumoren, Mammacarcinom, Ovarialcarcinom, Lebercarcinom, Magen- oder Pankreascarcinom. Die Herstellung solcher Antikörper erfolgt dabei nach den üblichen literaturbekannten Standardmethoden (z.B. Harlow, Lane: Antibodies, A Laboratory Manual; Cold Spring Harbor 1988).

Gegenstand der Erfindung ist somit schließlich die Verwendung der Verbindungen der Formel I, II, IV und V gemäß der Ansprüche 7 bis 11 zur Herstellung von Antikörpern für die Tumortherapie und die Tumordiagnostik.

Oben und unten werden folgende Abkürzungen verwendet:

| | |
|---|---|
| TCAI | Trichloracetimidat |
| OTBS | O-tert-Butyldimethylsilyl |
| TBAF | Tetrabutylammoniumfluorid |
| TMSOTf | Trimethylsilyltrifluormethansulfonsäureester |
| DBU | 1,8-Diazabicyclo [5.4.0] undec-7-en |
| OTDS | O-Thexyldimethylsilyl |

OTDPS O-tert-Butyldiphenylsilyl

S bedeutet einen Spacerrest $(CH_2)_qCOOR''$, worin R'' $C_1$ bis $C_4$-Alkyl bedeutet und q die angegebenen Werte annehmen kann. Alkyl kann dabei geradkettig oder verzweigt sein und bedeutet im einzelnen Methyl, Ethyl, n-Propyl, Isopropyl, n-Butyl, Isobutyl, sec. und tert. Butyl. Vorzugsweise bedeutet R'' Methyl oder Ethyl. q bedeutet vorzugsweise 6 bis 10, insbesondere aber 8. Der besonders bevorzugte Spacerrest ist $-(CH_2)_8COOEt$.

Z hat die angegebene Bedeutung, bedeutet aber vorzugsweise $OCH_2CH(NHCOC_{15}H_{31})CH(OH)-CH=CHC_{13}H_{27}$ (Ceramid). u kann die Werte 13 bis 23, Vorzugsweise 15 bis 20, insbesondere aber 15, und v die Werte 11 bis 17, vorzugsweise 13 bis 15, insbesondere aber 13, annehmen. W steht für eine sterisch anspruchsvolle (geräumige) Silylschutzgruppe, wie beispielsweise OTBS, OTDS oder OTDPS. Besonders bevorzugt ist OTBS. Ar bedeutet substituiertes oder unsubstituiertes Aryl oder Hetercaryl, vorzugsweise aber Phenyl. Die bevorzugte Schutzgruppe am jeweiligen Galactopyranosid-Ring ist also Benzyliden. Neben den erfindungsgemäßen Aryliden-Schutzgruppen ist auch, denn auch mit Einschränkungen, Isopropyliden verwendbar.

$R^{sg}$ steht für eine Schutzgruppe und bedeutet O-Acetyl, O-Benzyl oder O-Benzoyl.

Figur 1 (1.1 bis 1.5) stellt die wichtigtsten Verbindungen des erfindungsgemäßen Verfahrens dar. Die Ziffern beziehen sich auf die entsprechende Kennzeichnung in den Beispielen.

Im folgenden wird das erfindungsgemäße Verfahren in allgemeiner Form beschrieben. Verfahrensschritte, die Standardmethoden der Kohlenhydratchemie entsprechen, wie zum Beispiel die Einführung und Abspaltung geeigneter Schutzgruppen, sind nicht näher erläutert. Diesbezüglich wird auf die Standardliteratur verwiesen (z.B. T.W. Greene, Protective Groups in Organic Synthesis, 1981, John Wiley & Sons; Methods in Carbohydrate Chemistry, Vol. I-VIII, Academic Press .

Das erfindungsgemäße Verfahren arbeitet bevorzugt mit den oben und unten angegebenen Schutzgruppen (OAc, OBn, OBz, $N_3$, OPiv); es können jedoch auch andere bekannte in ihrer Wirkung auf Einführung und Abspaltung adäquate Schutzgruppen verwendet werden. Die Trichloracetimidat-Methode im allgemeinen und für die Synthese definierter Oligosaccharide ist in den oben erwähnten Literaturstellen hinreichend beschrieben worden. Details des erfindungsgemäßen Verfahrens sind den Beispielen zu entnehmen.

Ausgangsverbindungen für die erfindungsgemäßen Synthesen sind die Saccharide der Verbindungen der Formel II, aus denen erfindungsgemäß Donor- als auch Akzeptor-Bausteine (Formel V) hergestellt werden und aus denen die polymere Kette in sich wiederholenden Synthesezyklen aufgebaut wird. Kleinste Einheit sind die Azido-Trisaccharide der Formel II (k = 0, l = 0). Diese zentralen Basiselemente, bestehend aus einem Galactopyranose-, Fucopyranose- und einem Glucopyranose-Ring, werden erfindungsgemäß aus entsprechenden Monosaccharid-Bausteinen nach an sich bekannten Methoden hergestellt, wobei bevorzugt von leicht zugänglichem tert-Butyldimethyl-silyi-2-azido-4,6-O-benzyliden-glucopyranosid ausgegangen wird. Aber auch ein entsprechend anderweitig geschütztes 2-Azido-glucopyranosid ist geeignet. Einzelheiten sind in den Beispielen 1 bis 8 angegeben. Tabelle I (A,B) liefert eine Klassfizierung der Verbindungen der Formeln II und V, worin W exemplarisch OTBS bedeutet.

**Tab.I (A):**

Verbindungen des Typs II [Y/(x)]. Y = TCAI oder OTBS; x = Anzahl der Monosaccharid-Elemente; z = Anzahl Trisaccharid-Elemente; A = Nummer synthetisierte Verbindung (Bsp.); $R^{sg}$, k, l wie angegeben.

| Typ | x/z | $R^1$ | k | l | A |
|---|---|---|---|---|---|
| II [OTBS] | 3/1 | – | 0 | 0 | 8 |
| II [TCAI] | 3/1 | – | 0 | 0 | 10 |
| II [OTBS] | 6/2 | OH | 0 | 1 | 13 |
| II [TCAI] | 6/2 | $R^{sg}$ | 0 | 1 | 16 |
| II [OTBS][1] | 9/3 | OH | 1 | 1 | 38 |
| II [TCAI][1] | 9/3 | $R^{sg}$ | 1 | 1 | 41 |
| II [OTBS][1] | 12/4 | OH | 1 | 2 | 52 |
| II [TCAI][1] | 12/4 | $R^{sg}$ | 1 | 2 | 55 |
| II [OTBS][1] | 15/5 | OH | 1 | 3 | – |
| II [OTBS][1] | 18/6 | OH | 1 | 4 | – |
| II [OTBS][1] | 21/7 | OH | 1 | 5 | – |
| II [OTBS][1] | 24/8 | OH | 1 | 6 | – |
| II [OTBS][2] | 24/8 | OH | 6 | 1 | – |
| II [OTBS][3] | 24/8 | OH | 3 | 3 | – |

Die mit [1] bezeichneten Verbindungstypen beziehen sich auf solche Verbindungen, die aus einem Hexasaccharid-Donor und einem entsprechenden Akzeptor-Baustein herstellbar sind.

Wie in Tab. I dargelegt, sind auch Kombinationen von höherkettigen Donoren mit entsprechend niederkettigeren Akzeptor-Sacchariden möglich (z.B. [2]): hier wird ein 21-Saccharid-Donor mit einem Trisaccharid-Akzeptor gekoppelt; [3]): Kopplung zweier Dodecasaccharide).

**Tab.I (B):**

Verbindungen des Typs V [(y)]. y = Anzahl der Monosaccharid-Elemente; z = Anzahl Trisaccharid-Elemente; A = Nummer synthetisierte Verbindung (Beispiel), m wie angegeben.

| Typ | y/z | m | A |
|---|---|---|---|
| V [OTBS] | 3/1 | 0 | 12 |
| V [OTBS] | 6/2 | 1 | 51 |
| V [OTBS] | 9/3 | 2 | - |
| V [OTBS] | 12/4 | 3 | - |
| V [OTBS] | 15/5 | 4 | - |
| V [OTBS] | 18/6 | 5 | - |

Tabelle II stellt die Reaktionen verschiedener Verbindungen der Formel II (II [TCAI/OTBS (x)]) und der Formel V (V [OTBS (y)]) dar, worin W wiederum nur exemplarisch OTBS bedeutet.

**Tab. II:**

Die Werte in ( ) entsprechen der Anzahl der Monosaccharid-Einheiten in der jeweiligen Verbindung; A: Nummer synthetisierte Verbindung (Beispiel); B: Reaktionsbedingungen (Tab. III); C: zu erwartende Ausbeute (%) (± 5 %); X wie angegeben (Formel I).

| Edukt(e) ⟶ | Produkt(e) | A | B | C |
|---|---|---|---|---|
| II[OTBS(3)] | II[TCAI(3)] | 10 | a | 95 |
| II[OTBS(3)] | V[OTBS(3)] | 12 | b | 90 |
| II[TCAI(3)]+V[OTBS(3)] | II[OTBS(6)] | 13 | c | 80 |
| II[OTBS(6)] | II[TCAI(6)] | 16 | d | 95 |
| II[TCAI(6)] + III(2) | IV(8) | 26 | c | 75 |
| IV(8) | I[X(8)] | 27-37 | e-k | 75 |
| II[TCAI(6)]+V[OTBS(3)] | II[OTBS(9)] | 38 | c | 75 |
| II[OTBS(9)] | II[TCAI(9)] | 41 | d | 90 |
| II[TCAI(9)] + III(2) | IV(11) | 42 | c | 70 |
| IV(11) | I[X(11)] | 42-49 | e-k | 75 |
| II[OTBS(6)] | V[OTBS(6)] | 51 | b | 85 |
| II[TCAI(6)]+V[OTBS(6)] | II[OTBS(12)] | 52 | c | 80 |
| II[OTBS(12)] | II[TCAI(12)] | 55 | d | 95 |
| II[TCAI(12)] + III(2) | IV(14) | 56 | c | 75 |
| IV(14) | I[X(14)] | 57-63 | e-k | 75 |

**Tab. II (Fortsetzung):**

| Edukt(e) ⟶ | Produkt(e) | A | B | C |
|---|---|---|---|---|
| II[OTBS(9)] | V[OTBS(9)] | – | b | 85 |
| II[TCAI(9)]+V[OTBS(9)] | II[OTBS(18)] | – | c | 80 |
| II[OTBS(18)] | II[TCAI(18)] | – | d | 90 |
| II[TCAI(18)] + III(2) | IV(20) | – | c | 80 |
| IV(20) | I[X(20)] | – | e–k | 75 |
| II[OTBS(12)] | V[OTBS(12)] | – | b | 85 |
| II[TCAI(12)]+V[OTBS(12)] | II[OTBS(24)] | – | c | 75 |
| II[OTBS(24)] | II[TCAI(24)] | – | d | 90 |
| II[TCAI(24)] + III(2) | IV(26) | – | c | 70 |
| IV(26) | I[X(26)] | – | e–k | 75 |
| II[OTBS(9)] | V[OTBS(9)] | – | b | 85 |
| II[OTBS(15)] | V[OTBS(15)] | – | b | 80 |
| II[OTBS(18)] | V[OTBS(18)] | – | b | 80 |
| II[OTBS(15)] | II[OTBS(15)] | – | d | 95 |
| II[OTBS(21)] | II[OTBS(21)] | – | d | – |
| II[TCAI(9)]+V[OTBS(3)] | II[OTBS(12)] | – | c | 85 |
| II[TCAI(6)]+V[OTBS(9)] | II[OTBS(15)] | – | c | 85 |
| II[TCAI(9)]+V[OTBS(6)] | II[OTBS(15)] | – | c | 80 |
| II[TCAI(12)]+V[OTBS(3)] | II[OTBS(15)] | – | c | 85 |
| II[TCAI(6)]+V[OTBS(12)] | II[OTBS(18)] | – | c | 80 |
| II[TCAI(12)]+V[OTBS(6)] | II[OTBS(18)] | – | c | 80 |
| II[TCAI(15)]+V[OTBS(3)] | II[OTBS(18)] | – | c | 85 |
| II[TCAI(6)]+V[OTBS(15)] | II[OTBS(21)] | – | c | – |
| II[TCAI(9)]+V[OTBS(12)] | II[OTBS(21)] | – | c | – |
| II[TCAI(12)]+V[OTBS(9)] | II[OTBS(21)] | – | c | – |
| II[TCAI(15)]+V[OTBS(6)] | II[OTBS(21)] | – | c | – |
| II[TCAI(18)]+V[OTBS(3)] | II[OTBS(21)] | – | c | – |

| Edukt(e) ⟶ | Produkt(e) | A | B | C |
|---|---|---|---|---|
| II[TCAI(6)]+V[OTBS(18)] | II[OTBS(24)] | – | c | 80 |
| II[TCAI(9)]+V[OTBS(15)] | II[OTBS(24)] | – | c | 80 |
| II[TCAI(15)]+V[OTBS(9)] | II[OTBS(24)] | – | c | 80 |
| II[TCAI(18)]+V[OTBS(6)] | II[OTBS(24)] | – | c | 80 |
| II[TCAI(15)] + III(2) | IV(17) | – | c | 75 |
| II[TCAI(21)] + III(2) | IV(23) | – | c | – |
| IV(17) | I[X(17)] | – | e–k | 75 |
| IV(23) | I[X(23)] | – | e–k | 70 |

Tabelle III spezifiziert die in Tab. II angegebenen Reaktionsbedingungen. Dabei können die aufgeführten Reagenzien, sofern nichts anderes gesagt wird, insbesondere Lösungsmittel, Katalysatoren und Reduktionsmittel, durch entsprechendeandere in der Kohlenhydratchemie für diese Zwecke gängigen Equivalente ersetzt werden. Die Bedingungen gelten auch bei Einsatz von Verbindungen der Formel II und V, bei denen W nicht OTBS und Z nicht Ceramid (u = 15, v = 13) ist.

Vorzugsweise werden die einzelnen Reaktionsschritte, wie in der Tabelle spezifiziert und in den Beispielen detailliert geschildert, durchgeführt.

**Tab. III:**

| | Reaktionsschritt | Reagenzien/Bedingung |
|---|---|---|
| a | Einführung TCAI (1) | 1) TBAF;<br>2) $CCl_3CN$, DBU |
| b | Arylidenierung | 1) $NaOCH_3/CH_3OH$;<br>2) $ArCH(OCH_3)_2$, p-TsOH |
| c | Glycosidierung | $CH_3CN$, TMSOTf (-40 °C) |
| d | Einführung TCAI (2) | 1) $Ac_2O$/Pyridin;<br>2) TBAF, THF;<br>3) $CCl_3CN$, DBU |
| e | Überführung<br>N3 ⟶ HNAc | 1) $H_2S$, Pyridin, $H_2O$<br>2) $Ac_2O$/Pyridin |
| f | Peracetylierung | 1) Pd/C; $H_2$, $AcOH/CH_3OH$/Dioxan<br>2) $Ac_2O$/Pyridin |
| g | glycosidisches OH | $N_2H_4$ x AcOH |
| h | Einf. TCAI (Formel I) | wie a |
| i | Einführung Z, S | S: S-OH, TMSOTf, $CH_2Cl_2$<br>Z (Ceramid):<br>1) 2-Azido-3-benzoyloxy-4-octadecen-1-ol, $CH_2Cl_2$, TMSOTf;<br>2) $H_2S$, Pyridin, $H_2O$; (3-Dimethylaminopropyl)-N'-ethylcarbodiimid, $C_{15}H_{31}COOH$ |
| k | Entschützung | $NaOCH_3$, $CH_3OH$. |

Die Glycosidierungen (Reaktion c) werden in einer bevorzugten Ausführungsform in Nitrilen als Lösungsmittel, beispielsweise Acetonitril, durchgeführt, wodurch die Donorbausteine nicht mehr anomerenrein hergestellt zu werden brauchen. Dadurch ist in der Regel eine quantitative Synthese der Trichloracetimidate gegeben. Zweckmäßigerweise wird dem Ansatz ein Katalysator, beispielsweise Trimethylsilyltrifluormethansulfonat (TMSOTf) bei einer Reaktionstemperatur von -30 bis -80 °C, vorzugsweise - 40 °C,

zugesetzt.

Zur Herstellung der jeweiligen Trichloracetimidate (Reaktion a, d) sind nur sehr geringe Basenmengen (z. B. DBU, 0,1-1 %) notwendig, so daß eine nennenswerte Isomerisierung der Trichlor-acetimidate nicht eintritt.

Die Disaccharid-Verbindungen der Formel III, insbesondere Verbindung 25 lassen sich nach einer Vorschrift von Sato et al. (1988, Tetrahedron Lett. 29, 4097) leicht synthetisieren.

Hexasaccharid **13** (Beispiel 13) wird nach dem beschriebenen Verfahren in kristalliner Form erhalten, und ist somit in besonderer Weise geeigenet, als leicht isolierbare und quantitav reine Zwischenstufe für den Aufbau der polymeren Saccharidkette Verwendung zu finden.

Der Trichloracetimidat-Rest der Verbindungen der Formel I kann dann gewünschtenfalls ersetzt werden durch einen oben definierten Spacer S bzw. Lewis X-Antigenrest Z nach an sich bekannten Methoden (Reaktion i, k). Der Spacerrest S wird dabei vorzugsweise als S-OH bei Raumtemperatur unter katalytischer Wirkung von beispielsweise TMSOTf in Ausbeuten von 75 bis 90 % eingeführt. Der Rest Z wird vorzugsweise in zwei Schritten eingebracht und zwar, falls er Ceramid bedeutet, über die Verbindungen (2S,3R,4E)-2-Azido-3-benzoyloxy-4-octadecen-1-ol, Palmitinsäure und N-(3-Diaminopropyl)-N'-ethyl-carbodiimid(hydrochlorid).Nach vollständiger Entschützung des jeweiligen Polysaccharidteils erhält man die entsprechenden di- bis octameren Lewis X-Antigene der Formel I (n = 1 - 7).

Die folgenden Beispiele sollen die Erfindung in detaillierter Weise erläutern.

**Beispiel 1**

tert-Butyldimethylsilyl-2-azido-2-desoxy-β-D-glucopyranosid (1):

Verbindung 1 wird nach einer Vorschrift von Kinzy und Schmidt (Liebigs Ann. Chem. 1985, 1537-1545) dargestellt.

**Beispiel 2**

tert-Butyldimethylsilyl-2-azido-4,6-O-benzyliden-2-desoxy-β-D-glucopyranosid (2):

1 (14,37 g, 45 mmol) wird in absolutem Acetonitril (350 ml) mit Benzaldehyddimethylacetal (10,3 g, 67,5 mmol) und p-Toluolsulfonsäure (80 mg) versetzt. Nach einer Stunde bei Raumtemperatur wird wasserfreies Kaliumcarbonat (2 g) zugegeben und 30 min geschüttelt. Die Mischung wird filtriert und im Vakuum eingeengt. Flashchromatografie des Rückstandes [Petrolether/Essigsäuremethylester (5:1)] liefert 2 (17,06 g, 93 %) als farblosen Sirup. DC [Petrolether/Essigsäuremethylester (5:1)]: $R_f$ = 0,34.

**Beispiel 3**

2,3,4-Tri-O-benzyl-α/β-L-fucopyranose (3):

Verbindung 3 wird nach einer Vorschrift von Dejter-Juszynski und Flowers (Carbohydr. Res. 18 (1971) 219-226) dargestellt.

**Beispiel 4**

O-(2,3,4-Tri-O-benzyl-α/β-fucopyranosyl)-trichloracetimidat (4):

Zu einer Lösung von 3 (10,0 g, 23,mmol) in absolutem Dichlormethan (50 ml) wird Trichloracetonitril (10 g) und DBU (7 Tropfen) gegeben. Nach 30 min wird die leicht gelbliche Lösung im Vakuum eingeengt und mit einer kurzen Kieselgelsäule [Petrolether/Essigsäuremethylester (3:1) + 1 % Triethylamin] gereinigt. Man erhält 4 (11,85 g,89 %) im Verhältnis α:β = 1:4 als farbloses Öl, welches allmählich erstarrt; DC [Petrolether/Essigsäuremethylester (3:1) + 1 % Triethylamin]: $R_f$ = 0,76 für das α-Trichloracetimidat, $R_f$ = 0,50 für das β-Trichloracetimidat. Für das β-Trichloracetimidat ergeben sich folgende Werte: $[\alpha]^{20}$ = -16,0 (c = 1, $CHCl_3$).

**Beispiel 5**

tert- Butyldimethylsilyl-O-(2,3,4-tri-O-benzyl-α-L-fucopyranosyl)-(1→ 3)-2-azido-4,6-O-benzyliden-2-desoxy-β -D-glucopyranosid (5):

Verbindung 2 (2,93 g, 7,19 mmol) wird unter Argonatmosphäre in möglichst wenig abolutem Diethylether gelöst und mit 0.1 M Trimethylsilyltrifluormethansulfonatlösung (1 ml) versetzt.

Unter Rühren wird langsam eine konzentrierte Lösung von Verbindung 4 (6,5 g, 11,25 mmol) in absolutem Diethylether zugetropft. 5 min nach Beendigung der Zugabe des Trichloracetimidats wird mit Natriumhydrogencarbonat (ca. 0,5 g) neutralisiert, filtriert und im Vakuum eingeengt. Nach chromatographischer Reinigung mit Petrolether/Essigsäuremethylester (7:1) als Laufmittel erhält man Verbindung 5 (5,35 g, 85 %) als farblosen Schaum; DC [Petrolether/Essigsäuremethylester (5:1)]: $R_f$ = 0,54 $[\alpha]^{20}$ = -76,0 (c = 1, $CHCl_3$).

**Beispiel 6**

tert-Butyldimethylsilyl-O-(2,3,4-tri-O-benzyl-α-L-fucopyranosyl)-(1→ 3)-2-azido-6-O-benzyl-2-desoxy-β-D-glucopyranosid (6):

Eine Lösung von Verbindung 5 (10 g, 12,13 mmol) und Natriumcyanoborhydrid (7,6 g, 121 mmol) in absolutem Tetrahydrofuran (250 ml) wird mit Molekularsieb (4 Å) gerührt. Nun wird solange eine gesättigte Lösung von Chlorwasserstoff in Diethylether zugetropft, bis die Mischung sauer wird. Zur Aufarbeitung gibt man festes Natriumhydrogencarbonat, Diethylether (400 ml) sowie gesättigte Natriumhydrogencarbonatlösung (100 ml) in das Reaktionsgefäß und filtriert über Glaswolle ab. Die organische Phase wird abgetrennt, im Vakuum eingeengt und durch Flashchromatografie [Petrolether/Essigsäuremethylester (6:1→ 5,5:1)] gereinigt. Man erhält (6,72 g, 67 %) farblosen Schaum; DC (Petrolether/Essigsäuremethylester (4:1)]: $R_f$ = 0,48, $[\alpha]^{20}$ = -30,0 (c = 2, $CHCl_3$).

**Beispiel 7**

O-(2,3,4,6-Tetra-O-acetyl-α-D-galactopyranosyl)-trichloracetimidat (7):

Verbindung 7 wird nach einem Verfahren von Schmidt, Michel und Roos (Liebigs Ann. Chem. 1984, 1343-1357) dargestellt.

**Beispiel 8**

tert-Butyldimethylsilyl-O-(2,3,4,6-tetra-O-acetyl-β-D-galactopyranosyl)-(1→ 4)-[(2,3,4-tri-O-benzyl-α-L-fucopyranosyl)-(1→ 3)]-2-azido-6-O-benzyl-2-desoxy-β-D-glucopyranosid (8):

Akzeptor 6 (9,0 g, 10,89 mmol) und Trichloracetimidat 7 (10,72 g, 21,78 mmol) werden in möglichst wenig Diethylether gelöst. Unter Rühren wird eine Lösung von Trimethylsilyltrifluormethansulfonat (0,15 ml) in Diethylether (4 ml) zugetropft. Nach beendeter Zugabe des Katalysators wartet man noch 20 min, neutralisiert mit Natriumhydrogencarbonat (ca. 2 g), filtriert und engt im Vakuum ein. Flashchromatografie [Toluol/Aceton (16:1→ 15:1→ 14:1)] liefert Verbindung 8 (8,94 g, 71 %); DC [Toluol/Aceton (10:1)]: $R_f$ = 0,47, $[\alpha]^{20}$ = -19,0 (c = 1, $CHCl_3$).

**Beispiel 9**

2,3,4,6-Tetra-O-acetyl-β-D-galactopyranosyl-(1→ 4)-[(2,3,4-tri-O-benzyl-α-L-fucopyranosyl)-(1→ 3)]-2-azido-6-O-benzyl-2-desoxy-α/β-D-glucopyranose (9):

Zu einer Lösung der Verbindung 8 (5,0 g, 4,32 mmol) in absolutem Tetrahydrofuran (50 ml) gibt man bei -45 °C unter Rühren Eisessig (0,25 ml, 4,3 mmol) und tropft danach ein 1 M Lösung von Tetrabutylammoniumfluorid in Tetrahydrofuran (5 ml) zu.

Man erwärmt auf 0 °C, verdünnt mit Diethylether (500 ml) und wäscht die organische Phase mit gesättigter Natriumchloridlösung (3 × 80 ml). Einengen im Vakuum und anschließende Flashchromatografie [Petrolether/Essigsäuremethylester (5:4)] ergibt Verbindung 9 (4,32 g, 95 %) als farblosen Schaum; DC

[Petrolether/Essigsäuremethylester (5:4)]: $R_f$ = 0,36, $[\alpha]^{20}$ = -7,5 (c = 1, $CHCl_3$).

**Beispiel 10**

O-(2,3,4,6-Tetra-O-acetyl-β-D-galactopyranosyl)-(1→4)-[(2,3,4-tri-O-benzyl-α-L-fucopyranosyl)-(1→3)]-2-azido-6-O-benzyl-2-desoxy-α/β-D-glucopyranosyl-trichloracetimidat (10):

Zu einer Lösung von Verbindung 9 (2,19 g, 2,08 mmol) in absolutem Dichlormethan (100 ml) wird Trichloracetonitril (10 g) und DBU (drei Tropfen) gegeben. Nach 15 min wird im Vakuum eingeengt und über eine Kieselgelsäule mit Petrolether/Essigsäuremethylester (3:2) + 1 % Triethylamin als Laufmittel eluiert. Man erhält Verbindung 10 (2,47 g,100 %) im Verhältnis α:β = 4:5 als farblosen Schaum; $R_f$ = 0,36 für das α-Anomere, $R_f$ = 0,26 für das β-Anomere, $[\alpha]^{20}$ = +27,0 (c = 1, $CHCl_3$) für das α-Anomere, $[\alpha]^{20}$ = -24,0 (c = 1, $CHCl_3$) für das β-Anomere.

**Beispiel 11**

tert-Butyldimethylsilyl-O-β-D-galactopyranosyl-(1→4)-[2,3,4-tri-O-benzyl-α-L-fucopyranosyl)-(1→3)]-2-azido-6-O-benzyl-2-desoxy-β-D-glucopyranosid (11):

Verbindung 8 (4,2 g, 3,63 mmol) wird in absolutem Methanol (ca. 200 ml) gelöst und mit 2 M Natriummethanolatlösung (0,5 ml) versetzt. Man läßt die Lösung über Nacht stehen und neutralisiert am nächsten Tag mit Amberlite® IR 120 (Röhm u. Haas). Es wird vom Ionenaustauscher abfiltriert, im Vakuum eingeengt und zweimal mit Toluol coevaporiert. Verbindung 11 (3,22 g) wird quantitativ erhalten und ist ohne weitere Reinigung analysenrein; DC [Toluol/Aceton (1:1)]: $R_f$ = 0,6, $[\alpha]^{20}$ = -47,0 (c = 1, $CHCl_3$)

**Beispiel 12**

tert-Butyldimethylsilyl-O-(4,6-O-benzyliden-β-D-galactopyranosyl)-(1→4)-[2,3,4-tri-O-benzyl-α-L-fucopyranosyl)-(1→3)]-2-azido-6-O-benzyl-2-desoxy-β-D-glucopyranosid (12):

Zu einer Lösung von Verbindung 11 (3,5 g, 3,5 mmol) in absolutem Acetonitril (150 ml) gibt man Benzaldehyddimethylacetal (1,1 g, 7 mmol) und p-Toluolsulfonsäure (200 mg). Nach einer Stunde bei Raumtemperatur wird wasserfreies Kaliumcarbonat (ca. 2 g) zugegeben und 30 min geschüttelt. Es wird filtriert, im Vakuum eingeengt und über eine Kieselgelsäule mit Petrolether/Essigsäuremethylester (7:4→7:5→7:6→1:1) als Laufmittel eluiert. Verbindung 12 (3,22 g, 88 %) fällt als farbloser Schaum an; DC [Petrolether/Essigsäuremethylester (1:1)1: $R_f$ = 0,46, $[\alpha]^{20}$ = -96,0 (c = 1, $CHCl_3$).

**Beispiel 13**

tert-Butyldimethylsilyl-O-(2,3,4,6-tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→ 3)-(4,6-O-benzyliden-β-D-galactopyranosyl)-(1→4)-[(2,3,4-tri-O-benzyl-α-L-fucopyranosyl)-(1→ 3)]-2-azido-6-O-benzyl-2-desoxy-β-D-glucopyranosid (13):

Zu einer Lösung der Verbindung 10 (4,75 g, 4,0 mmol) und 12 (4,84 g, 4,5 mmol) in absolutem Acetonitril (60 ml) tropft man bei -40 °C eine 0,05 M Trimethylsilyltrifluormethansulfonatlösung (0,8 ml). Nach 10 min gibt man Natriumhydrogencarbonat (ca. 0,5 g) zu der Lösung und erwärmt auf Raumtemperatur. Nach Filtration und Einengen im Vakuum wird über eine kurze Kieselgelsäule mit Petrolether/Essigsäuremethylester (3:2) chromatografiert. Der beim Einengen entstehende Schaum wird in Diethylether (60 ml) gelöst und mit Petroleumbenzin (20 ml) zum Auskristallisieren gebracht. Man erhält farblose Kristalle (6,72 g, 80 %); DC [Toluol/Essigsäuremethylester (4:1)]: $R_f$ = 0,38, $[\alpha]^{20}$ = -56,5 (c = 1, $CHCl_3$), Schmp. = 195 °C.

**Beispiel 14**

tert-Butyldimethylsilyl-O-(2,3,4,6-tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→ 3)-(2-O-acetyl-4,6-O-benzyliden-β-D-galactopyranosyl)-(1→ 4)-[(2,3,4-tri-O-benzyl-α-L-fucopyranosyl)-(1→3)]-2-azido-6-O-benzyl-2-desoxy-β-D-glucopyranosid (14):

Verbindung 13 (6,20 g, 2,95 mmol) wird in Pyridin/Acetanhydrid (1:1, 30 ml) gelöst und über Nacht stehen gelassen Am nächsten Morgen wird im Vakuum eingeengt und zweimal mit Toluol coevaporiert. Flashchromatografie [Petrolether/Essigsäuremethylester (5:3)] liefert Verbindung 14 (6,13 g, 97 %); DC [Petrolether/Essigsäuremethylester (2:1)]: $R_f$ = 0,22, $[\alpha]^{20}$ = -70,5 (c = 1, $CHCl_3$).

**Beispiel 15**

O-(2,3,4,6-Tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-α/β-D-glucopyranose (15):

Eine Lösung der Verbindung 14 (5,83 g, 2,72 mmol) in absolutem Tetrahydrofuran (50 ml) wird, wie bei Verbindung 9 beschrieben, mit Eisessig (0,16 ml, 2,72 mmol) und einer 1 M Lösung von Tetrabutylammoniumfluorid in Tetrahydrofuran (3,4 ml) umgesetzt. Die Reinigung erfolgt mittels einer kurzen Kieselgelsäule mit Petrolether/Essigsäuremethylester (1:1→4:5) als Laufmittel. Man erhält Verbindung 15 (5,41 g, 98 %) als farblosen Schaum; DC [Petrolether/Essigsäuremethylester (1:1)]: $R_f$ = 0,30, $[\alpha]^{20}$ = -55,5 (c = 1, $CHCl_3$).

**Beispiel 16**

O-(2,3,4,6-Tetra-O-acetyl-β-D-galactopyranosyl)-(1→4)-[(2,3,4-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-α/β-D-glucopyranosyl-trichloracetimidat (16):

Zu einer Lösung der Verbindung 15 (5,41 g, 2,67 mmol) in absolutem Dichlormethan (150 ml) wird Trichloracetonitril (20 g) und DBU (5 Tropfen) gegeben. Nach 30 min wird im Vakuum eingeengt und mittels Flashchromatografie [Petrolether/Essigsäuremethylester (1:1) + 1 % Triethylamin] gereinigt. Man erhält Verbindung 16 (5,8 g, 100 %) im Verhältnis α:β = 1:2 als farblosen Schaum. DC [Petrolether/Essigsäuremethylester (1:1)]: $R_f$ = 0,56 für das α-Trichloracetimidat, $R_f$ = 0,62 für das β-Trichloracetimidat, $[\alpha]^{20}$ = -46,0 (c = 1, $CHCl_3$) für das α-Trichloracetimidat, $[\alpha]^{20}$ = -62,0 (c = 1, $CHCl_3$) für das β-Trichloracetimidat.

**Beispiel 17**

Benzyl-O-(2,4,6-tri-O-benzyl-β-D-galactopyranosyl)-(1→4)-3,6-di-O-benzyl-2-O-pivaloyl-β-D-glucopyranosid (25):

Verbindung 25 wird nach Sato et al. (Tetrahedron Lett. 29 (1988) 4097-4100) synthetisiert.

**Beispiel 18**

Benzyl-O-(2,3,4,6-tetra-O-acetyl-β-D-galactopyranosyl)-(1→4)-[(2,3,4-tri-O-benzyl-α-L-fucopyranosyl)-(1→3)]-(2-azid-6-O-benzyl-2-desoxy-β-D-gucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2,4,6-tri-O-benzyl-β-D-galactopyranosyl)-(1→4)-3,6-di-O-benzyl-2-O-pivaloyl-β-D-glucopyrnosid (26):

Bei einer Temperatur von -40 °C wird zu einer Lösung von Verbindung 16 als α/β Gemisch (1,35 g, 623 µmol) und dem Akzeptor 25 (1,21 g, 1,25 mmol) in Acetonitril (5 ml) eine 0,05 M Trimethylsilyltrifluormethansulfonatlösung (0,3 ml) getropft. Nach 15 min wird mit Natriumhydrogencarbonat (ca. 0,3 g) neutralisiert. Nachdem die Lösung auf Raumtemperatur gekommen ist, filtriert man vom Natriumhydrogencarbonat ab. Die Reinigung erfolgt mittels Flashchromatografie mit Petrolether/Essigsäuremethylester (2:1→3:2) als Laufmittel. Verbindung 26 (1,37 g, 74 %) wird als farbloser Schaum erhalten; DC [Petrolether/Essigsäure-methylester (3:2)]: $R_f$ = 0,42, $[\alpha]^{20}$ = -71,0 (c = 1, $CHCl_3$).

**Beispiel 19**

Benzyl-O-(2,3,4,6-tetra-O-acetyl-β-D-galactopyranosyl)-(1→4)-[(2,3,4-tri-O-benzyl-α-L-fucopyranosyl)-(1→3)]-(2-acetamido-6-O-benzyl-2-desoxy-β-D-glucopyranosyl)-(1→3)-(2-O-acetyl-4,6-O-benzyliden-β-D-galactopyranosyl)-(1→4)-[2,3,4-tri-O-benzyl-α-L-fucopyranosyl)-(1→3)]-(2-acetamido-6-O-benzyl-2-desoxy-β-D-glucopyranosyl)-(1→3)-(2,4,6-tri-O-benzyl-β-D-galactopyranosyl)-(1→4)-3,6-di-O-benzyl-2-O-pivaloyl-β-D-glucopyranosid (27):

Die Azidoverbindung 26 (1,37 g, 416 µmol) wird, in einem Pyridin/Wasser-Gemisch (ca. 100 ml) mit Schwefelwasserstoff 30 minuten lang umgesetzt und mehrere Tage lang bei Raumtemperatur stehen gelassen. Anschließende N-Acetylierung in Pyridin/Acetanhydrid (1:1) liefert nach Flashchromatografie [Toluol/Aceton (4:1→3:1)] einen farblosen Schaum (1,23 g, 88%; DC [Toluol/Aceton (5:2)]:$R_f = 0,43$, $[\alpha]^{20}$ = -44,0 (c = 1, $CHCl_3$).

**Beispiel 20**

Acetyl-O-(2,3,4,6-tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyransyl)-(1→3)-(2,4,6-tri-O-acetyl-β-D-galactopyranosyl)-(1→4)-3,6-di-O-acetyl-2-O-pivaloyl-α/β-D-glucopyranosid (28):

Verbindung 27 (1,34 g, 406 µmol) wird mit Palladiumkohle (10 % Pd, 800 mg) in einer 1:1:1-Mischung aus Essigsäure, Methanol und Dioxan (90 ml) unter einer Wasserstoffatmosphäre von 4 bar 48 h lang hydriert. Die Lösung wird von der Palladiumkohle abdekantiert und über Kieselgur filtriert. Die Palladiumkohle wird mehrmals in Methanol aufgeschlämmt und die Lösung erneut abdekantiert und filtriert. Anschließende Umsetzung in Pyridin/Acetanhydrid (1:1) liefert nach Flashchromatografie mit Toluol/Aceton/Methanol (11:6:0,3→10:6: 0,3) ein weißes Pulver (769 mg, 80 %); DC [Toluol/Aceton/Methanol (7:6:0,3)]: $R_f$ = 0,45, $[\alpha]^{20}$ = -35,0 (c = 1, $CHCl_3$).

**Beispiel 21**

2,3,4,6-Tetra-O-acetyl-β-D-galactopyranosyl-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D glucopyranosyl)-(1→3)-(2,4,6-tri-O-acetyl-β-D-galactopyranosyl)-(1→4)-3,6-di-O-acetyl-2-O-pivaloy-α/β-D-glucopyranose (29):

Die pracetylierte Verbindung 28 (758 mg, 320 µmol) wird in absolutem Dimethylformamid (20 ml) gelöst und bei 50 °C mit Hydrazinacetat (59 mg, 640 µmol) versetzt. Nach 30 min wird mit Essigsäureethylester (150 ml) verdünnt und mit gesättigter Natriumchloridlösung (30 ml) gewaschen. Die wäßrige Phase wird mit Essigsäureethylester (2 × 60 ml) reextrahiert. Die vereinigten organischen Extrakte werden im Vakuum eingeengt und mittels Flashchromatografie [Toluol/Methanol (10:1→9:1)] gereinigt. Man erhält Verbindung 29 (670 mg, 90 %) als weißes Pulver; DC [Toluol/Aceton/Methanol (6:6:0,3)]: $R_f$ = 0,47, $[\alpha]^{20}$ = -37,0 (c = 1, $CHCl_3$).

**Beispiel 22**

O-(2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-3,6-di-O-acetyl-2-O-pivaloyl-α-D-glucopyranosyl-trichloracetimidat (30):

Verbindung 29 (658 mg, 283 µmol) wird in absolutem Dichlormethan (20 ml) gelöst und mit Trichloracetonitril (1 ml) sowie DBU (1 Tropfen) versetzt. Nach 30 min wird die Mischung im Vakuum eingeengt und über eine kurze Kieselgelsäule mit Toluol/Methanol (10:1) als Laufmittel gereinigt. Man erhält ein weißes Pulver (608 mg, 87 %); DC [Toluol/Aceton/Methanol (7:6:0,3)]: $R_f$ = 0,48, $[\alpha]^{20}$ = -25,5 (c = 1, $CHCl_3$).

**Beispiel 23**

8-Ethoxycarbonyl-octan-1-ol (31):

Verbindung 31 wird aus Ölsäuremethylester nach einer Vorschrift von Gerlach et al. 1978 Heov. Chim. Acta, 61 (4), (1978) 1226) synthetisiert.

**Beispiel 24**

8-Ethoxycarbonyloctyl-O-(2,3,4,6-tetra-O-acetyl-β-D-galactopyranosyl-(1→4)-[(2,3,4-tri-O-acetyl-α-L-fucopyranosyl)-(1→ 3)]-(2-acetamido-6-O-acetyl-2-desoxy-β-D-glucopyranosyl)-(1→ 3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-3,6-di-O-acetyl-2-O-pivaloyl-β-D-glucopyranosid (32):

Trichloracetimidat 30 (233 mg, 94,5 µmol) und die als Spacer dienende Verbindungen nach Beispiel 23 (5 Tropfen, ca. 0,23 mmol) werden in absolutem Dichlormethan (2 ml) gelöst und mit Molekularsieb (4 Å) 3 h lang gerührt. Es wird eine 0,01 M Trimethylsilyltrifluormethansulfonatlösung (0,5 ml) zugetropft. Nach 30 min wird mit Natriumhydrogencarbonat neutralisiert, filtriert und im Vakuum eingeengt. Flashchromatografie [Toluol/Methanol (12:1→10:1→9:1)] liefert Verbindung 32 (175 mg, 75 %); DC [Toluol/Aceton/Methanol (7:6:0,3]: $R_f$ = 0,47, $[\alpha]^{20}$ = -48,0 (c = 1, $CHCl_3$).

**Beispiel 25**

8-Ethoxycarbonyloctyl-O-(β-D-galactopyranosyl-(1→4)-[(α-L-fucopyranosyl)-(1→3)]-(2-acetamido-2-desoxy-β-D-glucopyranosyl)-(1→3)-(β-D-galactopyranosyl)-(1→4)-[(α-L-fucopyranosyl)-(1→3)]-(2-acetamido-2-desoxy-β-D-glucopyranosyl)-(1→ 3)-(β-D-galactopyranosyl)-(1→4)-β-D-glucopyranosid (33):

Verbindung 32 (168 mg, 68,2 µmol) wird in absolutem Methanol (40 ml) gelöst, mit 1 M Natriummethanolatlösung (0,5 ml) versetzt und 24 h bei 45 °C gerührt. Nach Neutralisation mit Amberlite® IR 120 wird filtriert und im Vakuum eingeengt. Der Rückstand wird in absolutem Ethanol (100 ml) aufgenommen, mit 0,5 M Natriummethanolatlösung (1 ml) versetzt und bei 45 °C 24 h lang gerührt. Neutraliseren mit Amberlite® IR 120, Filtrieren und Einengen im Vakuum liefert eine nahezu farblose Verbindung. Die geringfügigen Verunreinigungen können durch eine kurze Flashsäule gepackt mit RP 18-Kieselgel mit Methanol/Wasser (1:1) als Laufmittel abgetrennt werden. Man erhält Ethoxycarbonyl-oct-1-yl-dimer-Lewis-X (105 mg, 90 %); DC [Essigsäuremethylester/Isopropanol/Wasser (3:3:2)]: $R_f$ = 0,43, RP 8-DC [Methanol/Wasser (1:1): $R_f$ = 0,37, $[\alpha]^{20}$ = -57,0 (c = 1, MeOH).

**Beispiel 26**

(2S,3R,4E)-2-Azido-3-benzoyloxy-4-octadecen-1-ol (34)

Verbindung 34 wird nach einer Vorschrift von Zimmermann (Tetrahedron Lett. 27 (1986) 481; Angew. Chem. 98 (1986) 722) synthetisiert.

**Beispiel 27**

O-(2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-(3,6-di-O-acetyl-2-O-pivaloyl-β-D-glucopyranosyl)-(1→1)-(2S,3R,4E)-2-azido-3-O-benzoyl-4-octadecen-1,3-diol (35):

Eine Lösung von Trichloracetimidat 30 (137 mg, 55,5 µmol) und Akzeptor 34 (48 mg, 111 µmol) in absolutem Dichlormethan (1 ml) wird, wie in Beispiel 24 beschrieben mit einer 0,01 M Trimethylsilyltrifluormethansulfonatlösung (0,3 ml) versetzt. Flashchromatografie [Toluol/Methanol (13:1→11:1) liefert Verbindung 35 (114 mg, 75 %) als weißes Pulver; DC [Toluol/Aceton/Methanol (7:6:0,3)]: $R_f$ = 0,55, $[\alpha]^{20}$ = -44,0 (c = 1, $CHCl_3$).

**Beispiel 28**

O-(2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-(3,6-di-O-acetyl-2-O-pivaloyl-β-D-glucopyranosyl)-(1→l)-(2S,3R,4E)-3-O-benzoyl-2-hexadecanamido-4-acetdecen-1,3-diol (36):

Schwefelwasserstoff wird durch eine Lösung von 34 (114 mg, 41,6 µmol) in Pyridin (10 ml) und Wasser (2 ml) geleitet. Nach 3 Tagen engt man bei 20 °C im Hochvakuum ein. Der feste Rückstand wird sofort in absolutem Dichlormethan (5 ml) gelöst und mit Palmitinsäure (25 mg, 100 µmol) sowie N-(3-Dimethylaminopropyl)-N'-ethylcarbodiimidhydrochlorid (E. Merck, Darmstadt, FRA) (29 mg, 150 µmol umgesetzt. Nach 18 h wird mit Dichlormethan (50 ml) verdünnt und mit Wasser (20 ml) gewaschen. Die organische Phase wird im Vakuum eingeengt. Flashchromatografie [Toluol/Methanol (12:1→11:1)] liefert 36 (105 mg, 85 %) als weißes Pulver; DC Toluol/Aceton/Methanol (7:6:0,3)]: $R_f$ = 0,55, $[\alpha]^{20}$ = -35,0 (c = 1, $CHCl_3$).

**Beispiel 29**

O-(β-D-Galactopyranosyl)-(1→4)-[(α-L-fucopyranosyl)-(1→ 3)]-(2-acetamido-2-desoxy-β-D-glucopyranosyl)-(1→3)-(β-D-galactopyranosyl)-(1→4)-[(α-L-fucopyranosyl)(1→3)]-(2-acetamido-2-desoxy-β-D-glucopyranosyl)-(1→3)-(β-D-galactopyranosyl)-(1→4)-(β-D-glucopyranosyl)-(1→1)-(2S,3R,4E)-2-hexadecanamido-4-octadecen-1,3-diol (37):

Zu einer Lösung von 36 (100 mg, 33,7 µmol) in absolutem Methanol (30 ml), gibt man 1 M Natriummethanolatlösung (0,5 ml) und rührt 24 h bei 45 °C. Es wird mit Amberlite® IR 120 neutralisiert, filtriert und im Vakuum eingeengt. Der Rückstand wird auf eine RP 18-Kieselgelsäule gegeben und mit Methanol/Wasser (12:1→14:1→16:1) eluiert. Das entschützte Glycosphingolipid 37 (62 mg, 90 %) wird in Wasser gelöst und lyophilisiert; DC [Essigsäuremethylester/Isopropanol/Wasser (7:6:4)]: $R_f$ = 0,5, RP 8-DC [Methanol/Wasser (9:1)]: $R_f$ = 0,33, $[\alpha]^{20}$ = -46,0 (c = 1, MeOH).

**Beispiel 30**

tert-Butyldimethylsilyl-O-(2,3,4,6-tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→ 3)]-(2-azido-6-O-benzyl-2-desoxy-β-D-glucopyranosyl)-(1→3)-(4,6-O-benzyliden-β-D-galactopyranosyl)-(1→4)- [(2,3,4-tri-O-benzyl-α-L-fucopyranosyl)-(1→3)]-2-azido-6-O-benzyl-2-desoxy-β-D-glucopyranosid (38):

Zu Trichloracetimidat 16 (1,72 g, 790 µmol) (Beispiel 16) und Akzeptor 12 (1,13 g, 1,05 mmol) (Beispiel 12) in absolutem Acetonitril (20 ), tropft man bei -40 °C eine 0,05 M Tri-methylsilyltrifluormethansulfonatlösung (0,6 ml). Es wird wie üblich aufgearbeitet. Flashchroamtografie [Toluol/Aceton (5:1→4:1)] liefert Verbindung 38 (1,73 g, 71 %); DC [Petrolether/Essigsäuremethylester (4:3)]: $R_f$ = 0,35, $[\alpha]^{20}$ = -83,0 (c = 1, $CHCl_3$).

**Beispiel 31**

tert-Butyldimethylsilyl-O-(2,3,4,6-tetra-O-acetyl-β-D-glactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-β-D-glucopyranosid (39):

Verbindung 38 (1,70 g, 551 µmol) wird 24 h mit Pyridin/Acetanhydrid (1:1, 10 ml) behandelt. Die Lösung wird im Vakuum eingeengt und mit Toluol coevaporiert. Flashchromatografie [Petrolether/Essigsäuremethylester (4:3)] liefert Verbindung 39 (1,62 g, 94 %); DC [Petrolether/Essigsäuremethylester (4:3): $R_f$ = 0,35, $[\alpha]^{20}$ = -84,0 (c = 1, $CHCl_3$).

**Beispiel 32**

O-(2,3,4,6-Tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-α/β-D-glucopyranose (40):

Eine Lösung der Verbindung 39 (1,40 g, 448 µmol) in absolutem Tetrahydrofuran (20 ml) wird, wie in Beispiel 9 beschrieben, mit Eisessig (26 µl, 448 µmol) und einer 1 M Lösung von Tetrabutylammoniumfluorid (1 ml) ungesetzt. Flashchromatografie [Petrolether/Essigsäuremethylester (4:5) liefert Verbindung 40 (1,23 g, 91 %); DC [Petrolether/Essigsäuremethylester (1:1)]: $R_f$ = 0.23, $[\alpha]^{20}$ = -73,0 (c = 1, $CHCl_3$).

**Beispiel 33**

O-(2,3,4,6-Tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-α/β-D-glucopyranose (41):

Zu einer Lösung der Verbindung 40 (980 mg, 325 µmol) in absolutem Dichlormethan (30 ml) wird Trichloracetonitril (3,0 ml) und DBU (1 Tropfen) gegeben. Nach 30 min engt man im Vakuum ein. Flashchromatografie [Toluol/Aceton (5:1) + 1 % Triethylamin] ergibt Trichloracetimidat 41 (943 mg, 92 %) im Verhältnsi α:β = 1:2; DC [Toluol/Aceton (5:1)]: $R_f$ = 0,22, für das α-Trichloracetimidat, $R_f$ = 0,29 für das β-Trichloracetimidat, $[\alpha]^{20}$ = -62,0 (c = 1, $CHCl_3$) für das α-Trichloracetimidat, $[\alpha]^{20}$ = -73,5 (c = 1, $CHCl_3$) für das β-Trichloracetimidat.

**Beispiel 34**

Benzyl-O-(2,3,4,6-tetra-O-acetyl-β-D-galactopyranosyl)-(1→ 4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2,4,6-tri-O-benzyl-β-D-galactopyranosyl)-(1→4)-3,6-di-O-benzyl-2-O-pivaloyl-β-D-glucopyranosid (42):

Trichloracetimidat 41 (662 mg, 210 µmol) und Akzeptor 25 (305 mg, 315 µmol) werden, wie in Beispiel 18 beschrieben, in Acetonitril (3 ml) bei -40 °C umgesetzt. Flashchromatografie [Toluol/Aceton (8:1→7:1)] liefert Verbindung 42 (598 mg, 72 %);DC [Toluol/Aceton (6:1)]: $R_f$ = 0,34, $[\alpha]^{20}$ = -84,0 (c = 1, $CHCl_3$).

**Beispiel 35**

Benzyl-O-(2,3,4,6-tetra-O-acetyl-β-D-galactopyranosyl)-(1→ 4)-[(2,3,4-tri-O-benzyl-α-L-fucopyranosyl)-(1→3)]-(2-acetamido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-acetamido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-acetamido-6-O-benzyl-2-desoxy-β-D-glucopyranosyl)-(1→3)-(2,4,6-tri-O-benzyl-β-D-galactopyranosyl)-(1→4)-3,6-di-O-benzyl-2-O-pivaloyl-β-D-glucopyranosid (43):

Durch eine Lösung von Verbindung 42 (591 mg, 149 µmol) in Pyridin (40 ml) und Wasser (14,5 ml) leitet man, wie in Beispiel 19 beschrieben, Schwefelwasserstoff. Anschließende N-Acetylierung in Pyridin/Acetanhydrid (1:1) liefert nach Flashchromatografie [Petroleter/Essigsäuremethylester (6:7→5:6→4:5)] Verbindung 43 (520 mg, 86 %); DC [Petrolether/Essigsäuremethylester (4:5): $R_f$ = 0,52, $[\alpha]^{20}$ = -84,0 (c = 1, $CHCl_3$).

**Beispiel 36**

Acetyl-O-(2,3,4,6-tetra-O-acetyl-β-D-galactopyranosyl)-(1→ 4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→ 40-3,6-di-O-acetyl-2-O-pivaloyl-α/β-D-glucopyranosid (44):

Verbindung 43 (346 mg, 85,5 µmol) wird, wie in Beispiel 20 beschrieben, mit Palladiumkohle (10 % Pd, 200 mg) in Essigsäure/Methanol/Dioxan (1:1:1, 30 ml) unter einer Wasserstoffatmosphäre von 4 bar 48 h lang hydriert. Anschließende Behandlung mit Pyridin/Acetanhydrid (1:1) ergibt nach Flashchromatografie [Toluol/Aceton/Methanol (9:6:0,3→8:6:0,3)] Verbindung 44 (214 mg, 78 %) als weißes Pulver; DC [Toluol/Aceton/Methanol (7:6:0,3)]: $R_f$ = 0,31, $[\alpha]^{20}$ = -45,0 (c = 1, $CHCl_3$).

**Beispiel 37**

2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→ 3)]-(2-acetamido-6-O-acetyl-2-desoxy-β-D-glucopyranosyl)-(1→3)-(2,4,6-tri-O-acetyl-β-D-galactopyranosyl)-(1→4)-3,6-di-O-acetyl-2-O-pivaloyl-α/β-D-glucopyranose (45):

Verbindung 44 (145 mg, 45,1 µmol) wird, wie in Beispiel 21 beschrieben, in absolutem Dimethylformamid (5 ml) mit Hydrazinacetat (9,2 mg, 100 µmol) umgesetzt. Es wird wie gewohnt aufgearbeitet. Flashchromatografie [Toluol/Aceton/Methanol (8,5:6:0,3→ 7,5:6:0,3)] ergibt Verbindung 45 (125 mg, 87 %); DC [$CH_2Cl_2$/MeOH (20:1): $R_f$ = 0,10, $[\alpha]^{20}$ = -48,0 (c = 1, $CHCl_3$).

**Beispiel 38**

O-(2,3,4,6-tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-3,6-di-O-acetyl-2-O-pivaloyl-α/β-D-glucopyranosyl-trichloracetimidat (46):

Eine Lösung der Verbindung 45 (114 mg, 35,8 µmol) in absolutem Dichlormethen (5 ml) wird mit Trichloracetonitril (1 ml) und DBU (1 Tropfen) versetzt. Nach 30 min wird im Vakuum eingeengt und mittels Flashchromatografie [$CH_2Cl_2$/MeOH (20:1→ 17,5:1→15:1)] gereinigt. Man erhält Verbindung 46 (109 mg, 89 %); DC [Toluol/Aceton/Methanol (7:6:0,3)]: $R_f$ = 0,31, $[\alpha]^{20}$ = -29,5 (c = 1, $CHCl_3$).

**Beispiel 39**

O-(2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyransid (43):osyl)-(1→4)-[(2,3,4-tri-O-acetyl-α-L-fucopyranosyl)-(1→ 3)]-(2-acetamido-6-O-acetyl-2-desoxy-β-D-glucopyranosyl)-(1→3)-(2,4,6-tri-O-acetyl-β-D-galactopyranosyl)-(1→4)-3,6-di-O-acetyl-2-O-pivaloyl-β-D-glucopyranosyl)-(1→ 1)-(2S,3R,4E)-2-azido-3-O-benzoyl-4-octadecen-1,3-diol (47):

Eine Lösung von Trichloracetimidat 46 (60,4 mg, 17,7 µmol) und Akzeptor 34 (15 mg, 35,4 µmol) in absolutem Dichlormethan (1 ml) wird mit Molekularsieb (4 Å) gerührt. Nach 4 h wird eine 5 mM Trimethylsilyltrifluormethansulfonatlösung (0,35 ml) zugetropft. Die Aufarbeitung erfolgt wie in Beispiel 24 beschrieben. Flashchromatografie [Toluol/Aceton/Methanol (10,5:6:0,4)] liefert Verbindung 47 (46,5 mg, 73 %); DC [Toluol/Aceton/Methanol (7:6:0,3)]: $R_f$ = 0,40, $[\alpha]^{20}$ = -53,0 (c = 1, $CHCl_3$).

**Beispiel 40**

O-(2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→ 3)]-(2-acetamido-6-O-acetyl-2-desoxy-β-D-glucopyranosyl)-(1→3)-(2,4,6-tri-O-acetyl-β-D-galactopyranosyl)-(1→4)-3,6-di-O-acetyl-2-O-pivaloyl-β-D-glucopyranosyl)-(1→1)-(2S,3R,4E)-3-O-benzoyl-2-hexadecanamido-4-octadecen-1,3-diol (48):

Schwefelwasserstoff wird durch eine Lösung von 47 (27,6 mg, 7,63 µmol) in Pyridin (10 ml) und Wasser (2 ml) geleitet. Nach 3 Tagen wird bei 20 °C im Hochvakuum eingeengt. Der Rückstand wird in absolutem Dichlormethan (2 ml) gelöst und mit Palmitinsäure (5 mg, 20 µmol) sowie N-(3-Dimethylaminopropyl)-N'-ethylcarbodiimidhydrochlorid (5 mg, 30 µmol) umgesetzt. Die Aufarbeitung erfolgt wie in Beispiel 28 beschrieben. Flashchromatografie [Toluol/Aceton/Methanol (10,5:6:0,3)] liefert Verbindung 48 (24,2 mg, 84 %); DC [Toluol/Aceton/Methanol (7:6:0,3)]: $R_f$ = 0,40, $[\alpha]^{20}$ = -45,0 (c = 1, $CHCl_3$).

**Beispiel 41**

O-(β-D-Galactopyranosyl)-(1→4)-[(α-L-fucopyranosyl)-(1→ 3)]-(2-acetamido-2-desoxy-β-D-glucopyranosyl)-(1→3)-(β-D-galactopyranosyl)-(1→4)-[α-L-fucopyranosyl)-(1→3)]-(2-acetamido-2-desoxy-β-D-glucopyranosyl)-(1→3)-(β-D-galactopyranosyl)-(1→4)-[(α-L-fucopyranosyl)-(1→3)]-(2-acetamido-2-desoxy-β-D-glucopyranosyl)-(1→3)-(β-D-galactopyranosyl)-(1→4)-(β-D-glucopyranosyl)-(1→1)-(2S,3R,4E)-2-hexadecanamido-4-octadecen-1,3-diol (49):

Zu einer Lösung von 48 (20.6 mg, 5,45 µmol) in absolutem Methanol (6 ml), gibt man 0,2 M Natriummethanolatlösung (0,25 ml) und rührt 24 h bei 45 °C. Es wird mit Amberlite® IR 120 neutralisiert, filtriert und im Vakuum eingeengt. Chromatografische Reinigung des Rückstandes mit RP 18-Kieselgel und Methanol/Wasser (9:1→11:1→12,5:1) liefert das trimere $Le^X$-Antigen (12,3 mg, 88 %); DC [Essigsäuremethylester/Isopropanol/Wasser (7:6:4)] $R_f$ = 0,23, RP 8-DC [Methanol/Wasser (10:1)]: $R_f$ = 0,42, $[\alpha]^{20}$ = -55,0 (c = 1, MeOH)

**Beispiel 42**

tert-Butyldimethylsilyl-O-(β-D-galactopyranosyl)-(1→4)-[(2,3,4-tri-O-benzyl-α-L-fucopyranosyl)-(1→3)-(2-azido-6-O-benzyl-2-desoxy-β-D-glucopyranolsyl)-(1→3)-(4,6-O-benzyliden-β-D-galactopyranosyl)-(1→4)-[-(2,3,4-tri-O-benzyl-α-L-fucopyranosyl)-(1→3)]-2-azido-6-O-benzyl-2-desoxy-β-D-glucopyranosid (50):

Verbindung 13 (819 mg, 390 µmol) (Beispiel 13) wird in absolutem Methanol (100 ml) suspendiert. Bei einer Temperatur von 45 °C gibt man eine 1,5 M Natriummethanolatlösung (10 Tropfen) dazu. Nach 2 h wird die klare Lösung mit Amberlite® IR 120 behandelt und filtriert. Einengen im Vakuum liefert Verbindung 50 (764 mg, 100 %); DC [Toluol/Aceton (5:3)]: $R_f$ = 0,37, $[\alpha]^{20}$ = -82,0 (c = 1, $CHCl_3$).

**Beispiel 43**

tert-Butyldimethylsilyl-O-(4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosid (51) :

Eine Lösung von Verbindung 50 (758 mg, 387 µmol) in absolutem Acetonitril (50 ml) wird, wie in Beispiel 12 beschrieben, mit Benzaldehyddimethylacetal (147 mg, 968 µmol) und p-Toluolsulfonsäure (30 mg) umgesetzt. Es wird wie üblich aufgearbeitet. Flashchromatografie [Petrolether/Essigsäuremethylester (3:2→ 5:4→1:1)] liefert Verbindung 51 (627 mg, 86 %); DE [Petrolether/Essigsäuremethylester(1:1)]: $R_f$ = 0,36, $[\alpha]^{20}$ = -112,0 (c = 1, $CHCl_3$).

**Beispiel 44**

tert-Butyldimethylsilyl-O-(2,3,4,6-tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-β-D-glucopyranosyl)-(1→3)-(4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-β-D-glucopyranosid (52):

Zu einer Lösung von Trichloracetimidat 16 (626 mg, 288 mmmol) und Akzeptor 51 (727 mg, 360 µmol) (Beispiel 43) in absolutem Acetonitril (12 ml), tropft m bei -40 °C eine 0,05 M Trimethylsilyltrifluormethansulfonatlösung (0,15 ml). Die Aufarbeitung erfolgt wie oben beschrieben. Flashchromatografie [Petrolether/Essigsäuremethylester (3:2→5:4)] liefert Verbindung 52 (859 mg, 77 %); DC [Petrolether/Essigsäuremethylester (1:1)]: $R_f$ = 0,63, $[\alpha]^{20}$ = -101.0 (c = 1, $CHCl_3$).

**Beispiel 45**

tert-Butyldimethylsilyl-O-(2,3,4,6-tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→ 3)-(2-O-acetyl-4,6-O-benzyliden-β-D-galactopyranosyl)-(1→ 4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-β-D-glucopyranosid (53):

Verbindung 52 (579 mg, 144 µmol) wird 24 h mit Pyridin/Acetanhydrid (1:1, 6 ml) behandelt. Es wird im Vakuum eingeengt und mit Toluol coevaporiert. Flashchromatografie [Petrolether/Essigsäuremethylester (3:2→5:4)] liefert Verbindung 53 (594 mg, 100 %); DC [Petrolether/Essigsäuremethylester (5:4)]: $R_f$ = 0,40, [α]20 = -97,0 (c = 1, $CHCl_3$).

**Beispiel 46**

O-(2,3,4,6-Tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-α/β-D-glucopyranose (54):

Eine Lösung von Verbindung 53 (582 mg, 141 µmol) in absolutem Tetrahydrofuran (10 ml) wird, wie in Beispiel 9 beschrieben, mit Eisessig (8 µl 141 µmol) und einer 1 M Lösung von Tetrabutylammoniumfluorid (0,3 ml) umgesetzt. Flashchromatografie [Petrolether/Essigsäuremethylester (1:1→5:6→4:5) liefert Verbindung 54 (531 mg, 94 %); DC [Petrolether/Essigsäuremethylester (4:5)]: $R_f$ = 0,58, $[\alpha]^{20}$ = -91,0 (c = 1, $CHCl_3$).

**Beispiel 47**

O-(2,3,4,6-Tetra-O-acetyl-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-2-azido-6-O-benzyl-2-desoxy-α/β-D-glucopyranosyl-trichloracetimidat (55):

Eine Lösung von Verbindung 54 (525 mg, 131 µmol) in absolutem Dichlormethan (20 ml) wird mit Trichloracetonitril (1,0 ml) und DBU 1 (1 Tropfen) versetzt. Nach 30 min wird im Vakuum eingeengt. Flashchromatografie [Toluol/Aceton (5:1→4:1) 1 % Triethylamin) liefert Verbindung 55 (495 mg, 91 %); als α,β-Gemisch ein Verhältnis α:β ≈ 1:2; $R_f$ = 0,47 (beide Anomere).

## Beispiel 48

Benzyl-O-(2,3,4,6-tetra-O-acetyl-β-D-galactopyranosyl)-(1→ 4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-azido-6-O-benzyl-2-desoxy-β-D-glucopyranosyl)-(1→ 3)-(2,4,6-tri-O-benzyl-β-D-galactopyranosyl)-(1→4)-3,6-di-O-benzyl-2-O-pivaloyl-β-D-glucopyranosid (56) :

Trichloracetimidat 55 (389 mg, 93,9 µmol) und Akzeptor 25 (136 mg, 141 µmol) werden, wie in Beispiel 18 beschrieben, in Acetonitril (2 ml) bei -40 °C umgesetzt. Flashchromatografie [Toluol/Aceton (9:1→7:1→6:1)] liefert Verbindung 56 (358 mg, 77 %); DC [Toluol/Aceton (5:1): $R_f$ = 0,37, $[\alpha]^{20}$ = -92,5 (c = 1, $CHCl_3$).

## Beispiel 49

Benzyl-O-(2,3,4,6-tetra-O-acetyl-β-D-glucopyranosyl)-(1→ 4)-[(2,3,4-tri-O-benzyl-α-L-fucopyranosyl)-(1→3)]-(2-acetamido-6-O-benzyl-2-desoxy-β-D-glucopyranosyl)-(1→3)-(2-O-acetyl-4,6-O-benzyliden-β-D-glucopyranosyl)-(1→4)-[(2,3,4-tri-O-benzyl-α-L-fucopyranosyl)-(1→3)]-(2-acetamido-6-O-benzyl-2-desoxy-β-D-glucopyranosyl)-(1→3)-(2-O-acetyl-4,6-O-benzyliden-β-D-glucopyranosyl)-(1→4)-[(2,3,4-tri-O-benzyl-α-L-fucopyranosyl)-(1→3)]-(2-acetamido-6-O-benzyl-2-desoxy-β-D-glucopyranosyl)-(1→3)-(2-O-acetyl-4,6-O-benzyliden-β-D-galactopyranosyl)-(1→4)-[(2,3,4-tri-O-benzyl-α-L-fucopyranosyl)-(1→3)]-(2-acetamido-6-O-benzyl-2-desoxy-β-D-glucopyranosyl)-(1→3)-(2,4,6-tri-O-benzyl-β-D-glucopyranosyl)-(1→ 4)-3,6-di-O-benzyl-2-O-pivaloyl-β-D-glucopyranosid (57):

Durch eine Lösung von Verbindung 56 (349 mg, 70,6 µmol) in Pyridin (40 ml) und Wasser (15 ml) leitet man, wie in Beispiel 19 beschrieben, Schwefelwasserstoff. Anschließende N-Acetylierung in Pyridin/Acetanhydrid (1:1) liefert nach Flashchromatografie [Petrolether/Essigsäuremethylester (4:5→3:4→2:3)] Verbindung 57 (305 mg, 85 %); DC [Petrolether/Essigsäuremethylester (3:4)]: $R_f$ = 0,26, $[\alpha]^{20}$ = -55,0 (c = 1, $CHCl_3$).

## Beispiel 50

Acetyl-O-(2,3,4,6-tetra-O-acetyl-β-D-glucopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-glucopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-2,4,6-tri-O-acetyl-β-D-glucopyranosyl)-(1→4)-[(2,3,4,-tri-O-aceyl-α-L-fucopyranosyl)-(1→3)]-(2-acetamido-6-O-acetyl-2-desoxy-β-D-glucopyranosyl)-(1→3)-(2,4,6-tri-O-acetyl-β-D-glucopyranosyl)-(1→4)-[(2,3,4-tri-O-acetyl-α-L-fucopyranosyl)-(1→ 3)]-(2-acetamido-6-O-acetyl-2-desoxy-β-D-glucopyranosyl)-(1→ 3)-(2,4,6-tri-O-actyl-β-D-glucopyranosyl)-(1→4)-3,6-di-O-acetyl-2-O-pivaloyl-α/β-D-glucopyranosid (58):

Verbindung 57 (300 mg, 59,0 µmol) wird, wie in Beispiel 20 beschrieben, mit Palladiumkohle (10 % Pd, 150 mg) in Essigsäure/Methanol/Dioxan (1:1:1, 30 ml) unter einer Wasserstoffatmosphäre von 4 bar 48 h lang hydriert. Anschließende Behandlung mit Pyridin/Acetanhydrid (1:1) ergibt nach Flashchromatografie [Dichlormethan/Methanol (97:3→96:4→95:5→ 94:6)] Verbindung 58 (193 mg, 81 %); DC [Toluol/Aceton/Methanol (6:6:0,3)]: $R_f$ = 0,41, [α]20 = -48,0 (c = 1, $CHCl_3$).

**Beispiel 51**

2,3,4,6-Tetra-O-acetyl-β-D-glucopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4.6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-glucopyranosyl)-(1→4)-[(2,3,4-tri-O-acetyl-α-L-fucopyranosyl)-(1→3)]-(2-acetamido-6-O-acetyl-2-desoxy-β-D-glucopyranosly)-(1→3)-(2,4,6-tri-O-actyl-β-D-galactopyranosyl)-(1→4)-3,6-di-O-acetyl-2-O-pivaloyl-α/β-D-glucopyranosid (59):

Verbindung 58 (186 mg, 46,0 µmol) wird, wie in Beispiel 21 beschrieben, in absolutem Dimethylformamid (6 ml) mit Hydrazinacetat (9,2 mg, 100 µmol) umgesetzt. Es wird wie gewohnt aufgearbeitet. Flashchromatografie [Dichlormethan/Methanol (25:1→20:1→15:1)] liefert Verbindung 59 (163 mg, 88 %); DC ($CH_2Cl_2$/MeOH (19:1): $R_f$ = 0,12, $[\alpha]^{20}$ = -53,5 (c = 1, $CHCl_3$).

**Beispiel 52**

O-(2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-deoxy-β-D-glucopyranosyl)-(1→3)-(2,4,6-tri-O-acetyl-β-D-galactopyranosyl)-(1→4)-[(2,3,4-tri-O-actyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→ 4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosy)-(1→4)-3,6-di-O-acetyl-2-O-pivaloyl-α/β-D-glucopyranosyl-trichloractimidat (60):

Zu einer Lösung von Verbindung 59 (156 mg, 38,8 µmol) in absolutem Dichlormethan (6 ml) wird Trichloracetonitril (1 ml) und DBU (1 Tropfen gegeben. Nach 30 min wird im Vakuum eingeengt und mittels Flashchromatografie [Dichlormethan/Methanol (20:1→17,5→15:1)] gereinigt. Man erhält Verbindung 60 (141 mg, 89 %) als α/β-Gemisch im Verhältnis α:β = 3:1; DC [Dichlormethan/Methanol (16:1)]: $R_f$ = 0,31 (beide Anomere).

**Beispiel 53**

O-(2,3,4,6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-actyl-β-D-galactopyranosyl)-(1→4)-(3,6-di-O-acetyl-2-O-pivaloyl-β-D-glucopyranosyl)-(1→1)-(2S,3R,4E)-2-azido-3-O-benzoyl-4-octadecen-1,3-diol (61):

Eine Lösung von Trichloracetimidat 60 (134 mg, 32,8 µmol) und Akzeptor 34 (28,2 mg 65,6 µmol) in absolutem Dichlormethan (2 ml wird mit Molekularsieb (4 Å) gerührt. Nach 6 h wird eine 0,01 M Trimethylsilyltrifluormethansulfonatlösung (0,33 ml) zugetropft. Die Aufarbeitung erfolgt wie in Beispiel 24 beschrieben. Flashchromatografie [Dichlormethan/Methanol (25:1→22,5:1→20:1)] liefert Verbindung 61 (110 mg, 76 %); DC [Toluol/Aceton/Methanol (7:6:0,3)]: $R_f$ = 0,45, $[\alpha]^{20}$ = -54,0 (c = 1, $CHCl_3$).

**Beispiel 54**

O-(2,3,4-6-Tetra-O-acetyl-β-D-galactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[-(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-[(2,3,4-tri-O-actyl-α-L-fucopyranosyl)-(1→3)]-(2-acetamido-6-O-acetyl-2-desoxy-β-D-glucopyranosyl)-(1→3)-(2,4,6-tri-O-acetyl-β-D-glactopyranosyl)-(1→4)-[(2,3,4-tri-O-acetyl-α-L-fucopyranosyl)-(1→3)]-(2-acetamido-6-O-acetyl-2-desoxy-β-D-glucopyranosyl)-(1→3)-(2,4,6-tri-O-acetyl-β-D-galactopyranosyl)-(1→4)-(3,6-di-O-acetyl-2-O-pivaloyl-β-D-glucopyranosyl)-(1→1)-(2S,3R,4E)-3-O-benzoyl-2-hexandecanamido-4-octadecen-1,3-diol (62):

Durch eine Lösung von 61 (104 mg, 23,7 µmol) in Pyridin (10 ml) und Wasser (2 ml) wird Schwefelwasserstoff geleitet. nach 3 Tagen wird bei 20 °C im Hochvakuum eingeengt. Der Rückstand wird sofort in absolutem Dichlormethan (5 ml) gelöst und mit Palmitinsäure (20 mg, 80 µmol) sowie N-(3-Dimethylaminopropyl)-N'-ethylcarbodiimidhydrochlorid (23 mg, 120 µmol) umgesetzt. Die Aufarbeitung erfolgt wie in Beispiel 28 beschrieben. Nach Flashchromatografie [Dichlormethan/Methanol (25:1→23:1→20:1)] erhält man Verbindung 62 (92,3 mg, 85 %); DC [Toluol/Aceton/Methanol (6:7:0,3)]: $R_f$ = 0,64, $[\alpha]^{20}$ = -47,0 (c = 1, $CHCl_3$).

**Beispiel 55**

O-(β-D-Galactopyranosyl)-(1→4)-[α-L-fucopyranosyl)-(1→3)]-(2-acetamido-2-desoxy-β-D-glucopyranosyl)-(1→3)-(β-D-galactopyranosyl)-(1→4)-[(α-L-fucopyranosyl)-(1→3)]-(2,acetamido-2-desoxy-β-D-glucopyranosyl)-(1→3)-(β-D-galactopyranosyl)-(1→ 4)-[(α-L-fucopyranosyl)-(1→3)]-(2-acetamido-2-desoxy-β-D-glucopyranosyl)-(1→3)-(β-D-galactopyranosyl)-(1→4)-[(α-L-fucopyranosyl)-(1→3)]-(2-acetamido-2-desoxy-β-D-glucopyranosyl)-(1→3)-(β-D-galactopyranosyl)-(1→4)-(β-D-glucopyranosyl)-(1→1)-(2S,3R,4E)-2-hexadecanamido-4-octadecen-1,3-diol (63):

Zu einer Lösung von 62 (87,5 mg, 19,1 µmol) in absolutem Methanol (30 ml), gibt man 1 M Natriummethanolatlösung (0,25 ml) und rührt 24 h bei 45 °C. Es wird mit Amberlite® IR 120 neutralisiert, filtriert und im Vakuum eingeengt. Chromatografische Reinigung des Rückstandes mit RP 18-Kieselgel und Methanol/Wasser (8:1→12:1) → Methanol liefert das tetramere $Le^x$-Antigen (54,2 mg, 91 %); DC [Dichlormethan/Methanol/Wasser (7:7:2)] $R_f$ = 0,35, RP 8-DC [Methanol/Wasser (9:1)]: $R_f$ = 0,32, $[\alpha]^{20}$ = -57,5 (c = 1, MeoH)

## Patentansprüche

1. Verfahren zur Herstellung von Polaren Lewis X-Sacchariden der Formel I,

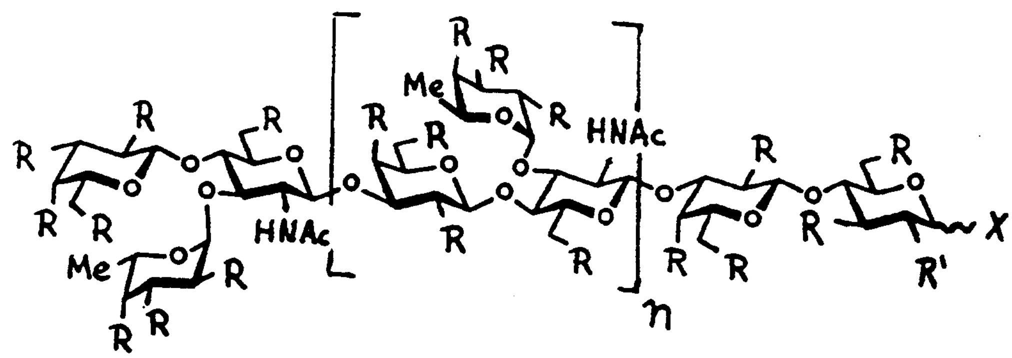

worin

| | |
|---|---|
| X | OC(=NH)$CCl_3$ (TCAI), Z oder S, |
| Z | $OCH_2CH(NHCOC_uH_{2u+1})CH(OH)CH=CHC_vH_{2v+1}$, |
| S | $(CH_2)_qCOOR''$, |
| R | OH, $R^{sg}$, |
| $R^{sg}$ | OAc, OBn oder OBz, |
| R' | OH, OAc oder OPiv, |
| R'' | $C_1$ bis $C_4$-Alkyl, |
| Ac | Acetyl, |
| Bn | Benzyl, |
| Bz | Benzoyl, |
| Piv | Pivaloyl, |
| n | eine ganze Zahl von 0 bis 7, |
| q | eine ganze Zahl von 4 bis 12, |
| u | eine ganze Zahl von 13 bis 23 und |
| v | eine ganze Zahl von 11 bis 17, |

bedeuten, ausgehend von einem Hexasaccharid-Baustein, dadurch gekennzeichnet, daß folgende

Schritte durchgeführt werden:

(a) Glycosidierung eines an der 1-O-Position aktivierten Donorsaccharid-Bausteins (II [TCAI/(x)]) ausgewählt aus einer der Verbindungen der Formel II,

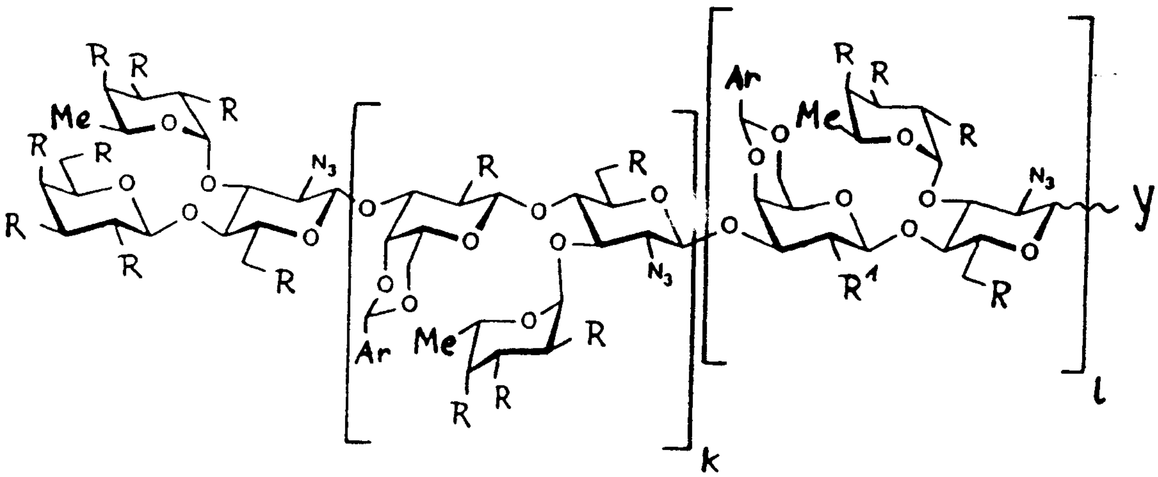

worin

| | |
|---|---|
| Y | OC(=NH)$CCl_3$ (TCAI) oder W, |
| W | O-tert-Butyldimethylsilyl (OTBS), O-Thexyldimethylsilyl (OTDS) oder O-tert-Butyldiphenylsilyl (OTDPS), |
| R | wie angegeben, |
| $R^1$ | R, |
| Ar | Aryl, |
| Me | Methyl, |
| k, l | eine ganze Zahl von 0 bis 6 |

bedeuten,

wobei, wenn Y TCAI bedeutet, $R^1$ OAc, OBn oder OBz ist, und wenn Y W bedeutet, $R^1$ OH ist, mit einem Akzeptorsaccharid-Baustein der Formel V ( V [(y)] ),

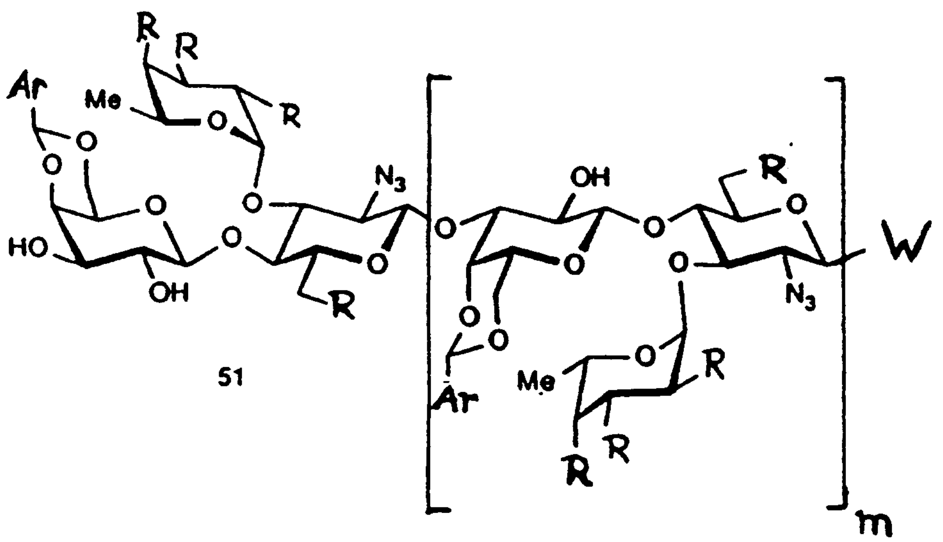

51

I

worin R, Ar, Me und W die angegebenen Bedeutungen haben und m eine ganze Zahl von 0 bis 5 ist,

zu einem höherkettigen Saccharid der Formel II (II [W (x + y)], wobei x 6, 9, 12, 15, 18, 21 und y 3, 6, 9, 12, 15 und 18 bedeuten und die Anzahl der Monosaccharid-Elemente in dem betreffenden Polysaccharid wiedergeben, und x + y nicht größer als 24 sein dürfen;

(b) Umsetzung in das entsprechende höherkettige Donorsaccharid der Formel II ( II [TCAI/(x + y)] ) durch Substitution der freien OH-Gruppe(n) durch eine entsprechende Schutzgruppe und des W-Restes durch den TCAI-Rest;
(c) Glycosidierung des an der 1-O-Position aktivierten Donorsaccharids aus (b) mit einem als Akzeptor wirksamen Disaccharids der Formel III,

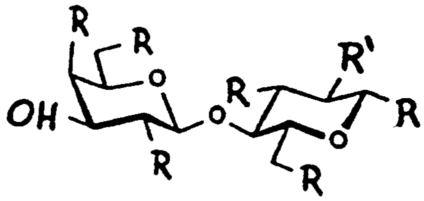

III

worin R und R' die angegebenen Bedeutungen haben, zu einem Saccharid der Formel IV [(x + y + 2)],

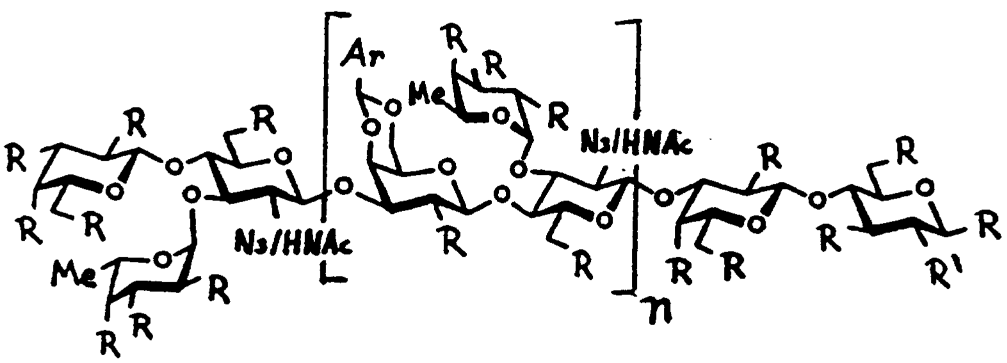

IV

worin R, R', Ar, Me und n die angegebenen Bedeutungen haben; und
(d) Reduktion der Azidogruppen zu Acetamidogruppen der Verbindung IV, Abspaltung der Aryliden- und Benzyl-Reste und Peracetylierung der Reste R und R', Substitution der glycosidischen Acetylgruppe durch OH und Umsetzung zum entsprechenden Trichloracetimidat (X = TCAI) der Formel I und gewünschtenfalls Modifizierung des Restes X zu Z oder S und Entfernung der Schutzgruppen (R, R' = OH).

**2.** Verfahren nach Anspruch 1, dadurch gekennzeichnet, daß Verbindungen der Formel II eingesetzt werden, bei denen der endständige Galactopyranose-Ring acetyliert ist.

**3.** Verfahren nach Anspruch 1 oder 2, dadurch gekennzeichnet, daß man eine pivaloylierte Verbindung der Formel III (R' = OPiv) einsetzt.

**4.** Verfahren nach einem der Ansprüche 1 bis 3, dadurch gekennzeichnet, daß die Glycosidierungen in Nitrilen als Lösungsmittel durchgeführt werden.

**5.** Verfahren nach einem der Ansprüche 1 bis 4, dadurch gekennzeichnet, daß man als Hexasaccharid-Baustein Verbindung 13 (FIG. 1 (1.1)) einsetzt.

**6.** Verfahren nach einem der Ansprüche 1 bis 5, dadurch gekennzeichnet, daß entsprechende Verbindungen eingesetzt werden, bei denen W O-tert-Butyldimethylsilyl (OTBS), Ar Phenyl, u = 15 und v = 13 bedeutet.

**7.** Polymere Lewis X-Saccharide der Formel I mit den in Anspruch 1 angegebenen Bedeutungen, wobei jedoch n eine ganze Zahl von 2 bis 7 bedeutet mit der Maßgabe, daß, wenn n 2 bedeutet, X nicht Z und R, R' nicht OH sein dürfen.

8. Tetrameres Lewis X-Antigen der Formel I (n = 3); Hexameres Lewis X-Antigen der Formel I (n = 5); Octameres Lewis X-Antigen der Formel I (n = 7).

9. Polymere Lewis X-Saccharide der Formel II mit den in Anspruch 1 angegebenen Bedeutungen, wobei jedoch k 1 ist und l eine ganze Zahl von 1 bis 5 bedeutet.

10. Polymere Lewis X-Saccharide der Formel IV mit den in Anspruch 1 angegebenen Bedeutungen, wobei jedoch n eine ganze Zahl von 2 bis 7 bedeutet.

11. Polymere Lewis X-Saccharide der Formel V mit den in Anspruch 1 angegebenen Bedeutungen, wobei jedoch m eine ganze Zahl von 1 bis 5 bedeutet.

12. Verfahren zur Herstellung von hexameren Lewis X-Sacchariden der Formel II (II [TCAl/W/(x)]), k = 0, l = 1, x = 6), dadurch gekennzeichnet, daß man ein mit einer sterisch anspruchsvollen Silylschutzgruppe ausgestattetes 2-Azido-Aryliden-glucopyranosid mit Trichloracetimidaten entsprechender Monosaccharide zu einem Trisaccharid der Formel II ( II [W/(x)], k = 0, l = 0, x = 3) umsetzt, aus diesem ein Donor-Trisaccharid der Formel II ( II [TCAl/(x)], x = 3) und ein Akzeptor-Trisaccharid der Formel V ( V [(y)], m = 0, y = 3) herstellt, welche miteinander zu dem entsprechenden Hexasaccharid umgesetzt werden.

13. Verfahren nach Anspruch 12, dadurch gekennzeichnet, daß man tert-Butyldimethylsilyl-2-azido-4,6-O-benzyliden-glucopyranosid einsetzt.

14. Verfahren zur Herstellung von Lewis X - Sacchariden der Formel IV, dadurch gekennzeichnet, daß man ein Donorsaccharid der Formel II ( II [TCAl/(x)] ) gemäß Anspruch 1 (c) umsetzt.

15. Verfahren zur Herstellung von Lewis X - Sacchariden der Formel V, dadurch gekennzeichnet, daß man die Schutzgruppen des endständigen Galactopyranose-Rings der Verbindung der Formel II ( II [W/(x)], k = 0, l = 0, ) abspaltet und an den Positionen 4 und 6 arylideniert, vorzugsweise benzylideniert.

16. Verfahren zur Herstellung von Lewis X-Sacchariden der Formeln I, IV und V nach einem der Ansprüche 1 bis 6 oder 14 bis 15, dadurch gekennzeichnet, daß man Hexasaccharide der Formel II einsetzt, welche nach einem Verfahren gemäß Anspruch 12 hergestellt wurden.

17. Verwendung der Verbindungen der Formeln I, II, IV und V gemäß der Ansprüche 7 bis 11 zur Herstellung von Antikörpern für die Tumortherapie und Tumordiagnostik.

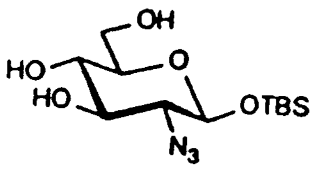

1

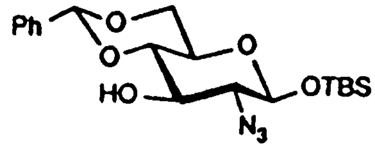

2

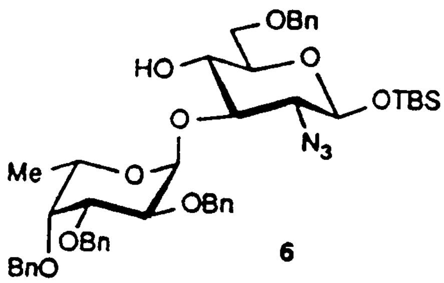

6

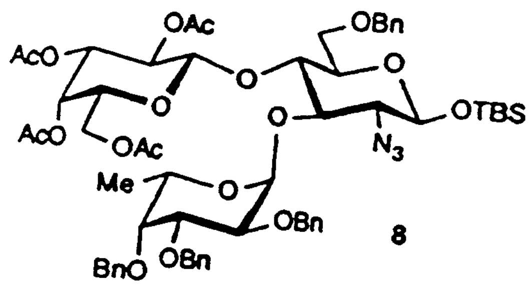

8

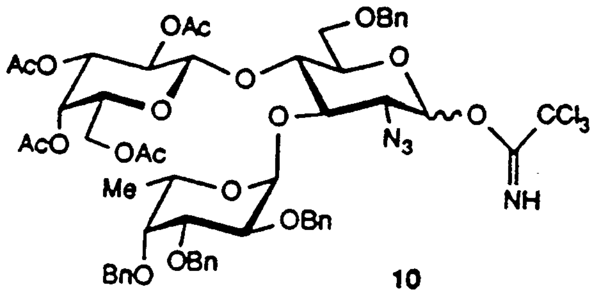

10

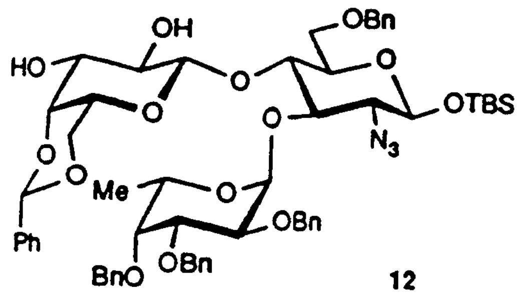

12

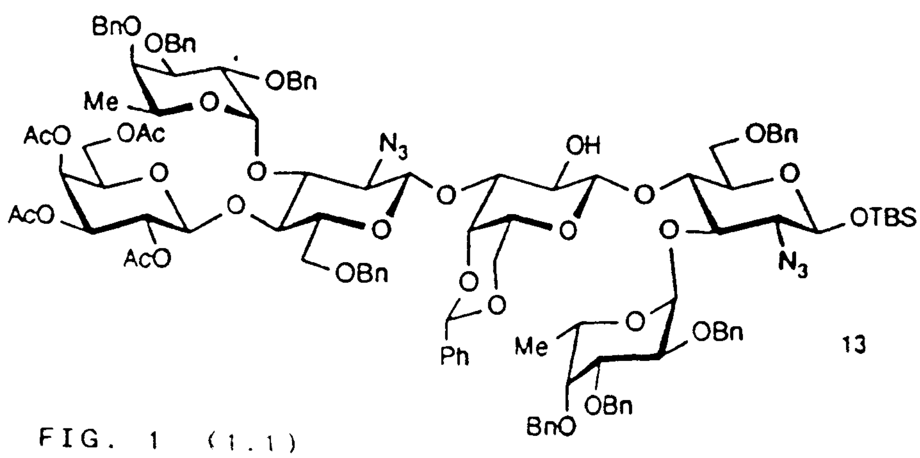

13

FIG. 1 (1.1)

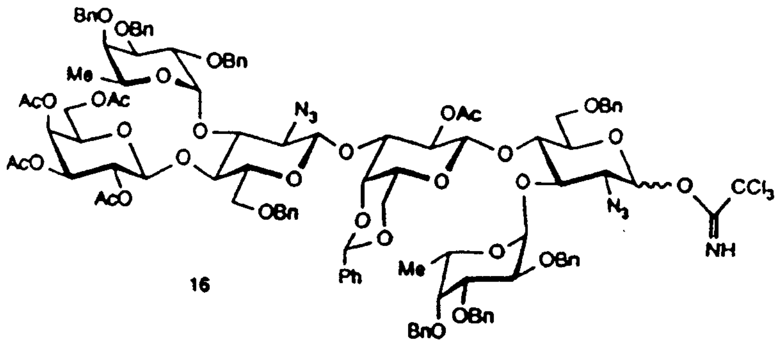

16

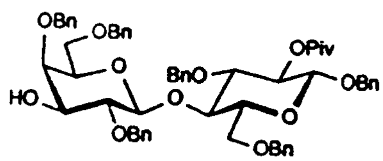

25

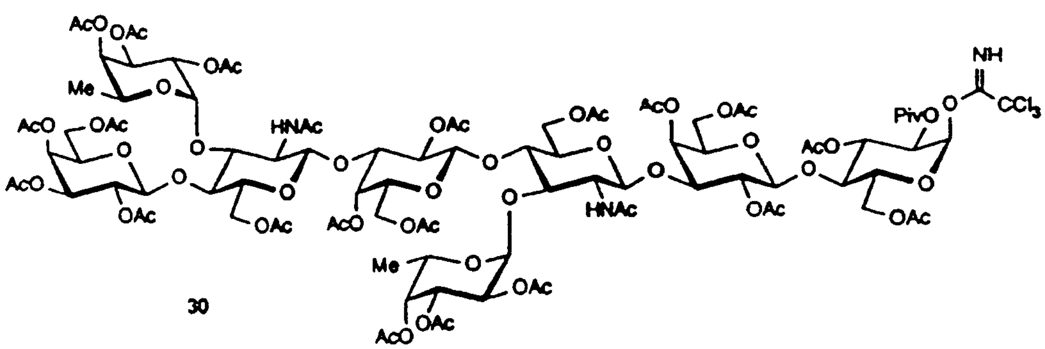

30

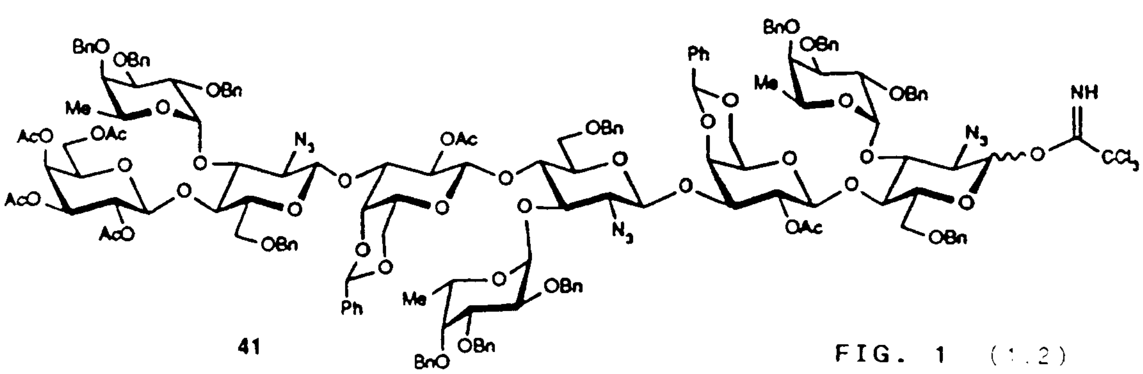

41

FIG. 1 (1.2)

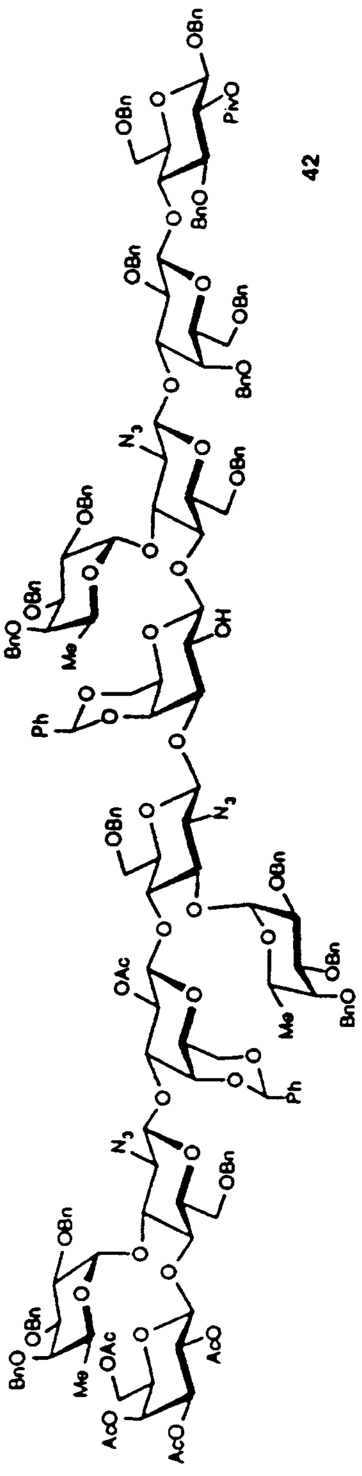

42

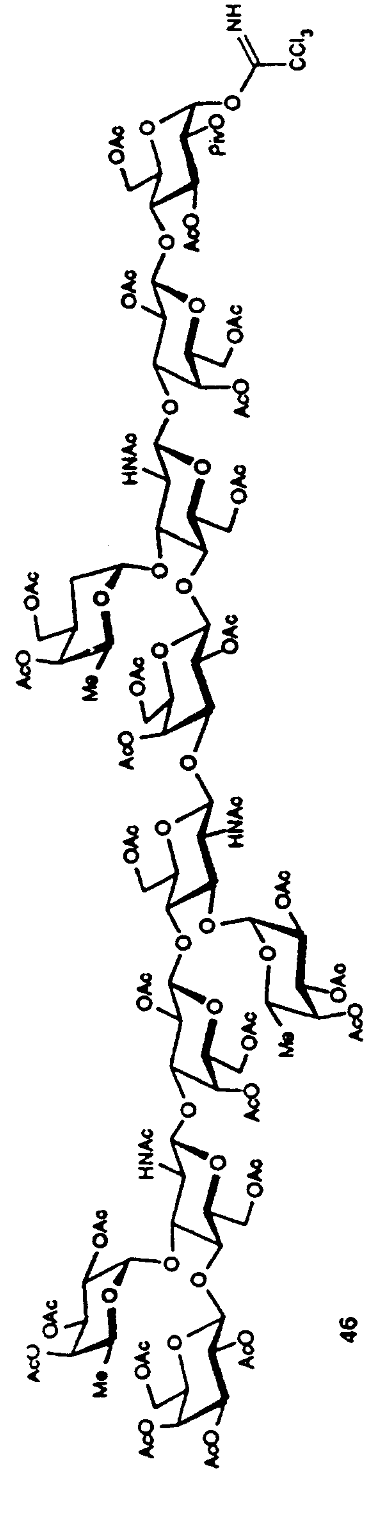

46

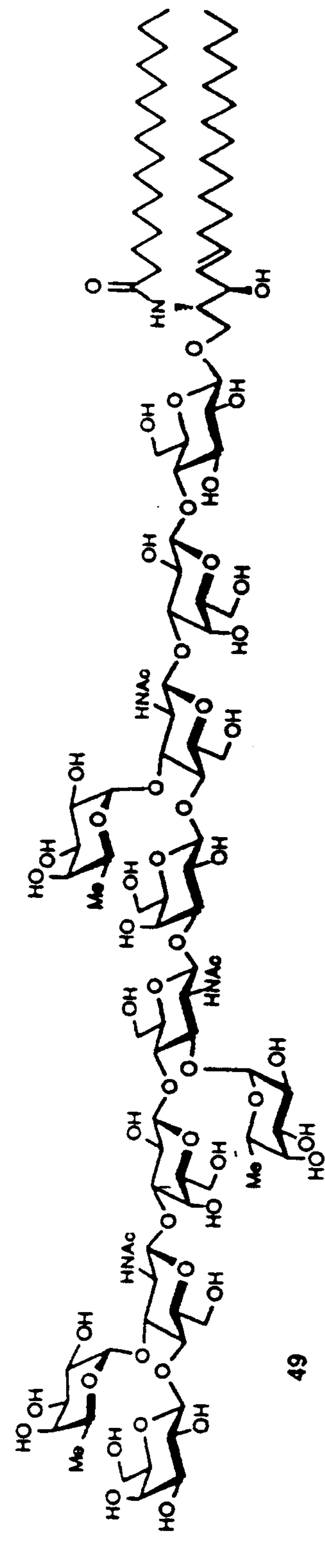

49

FIG. 1 (1.3)

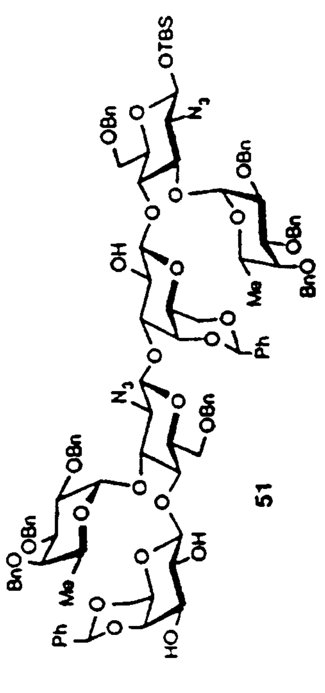

51

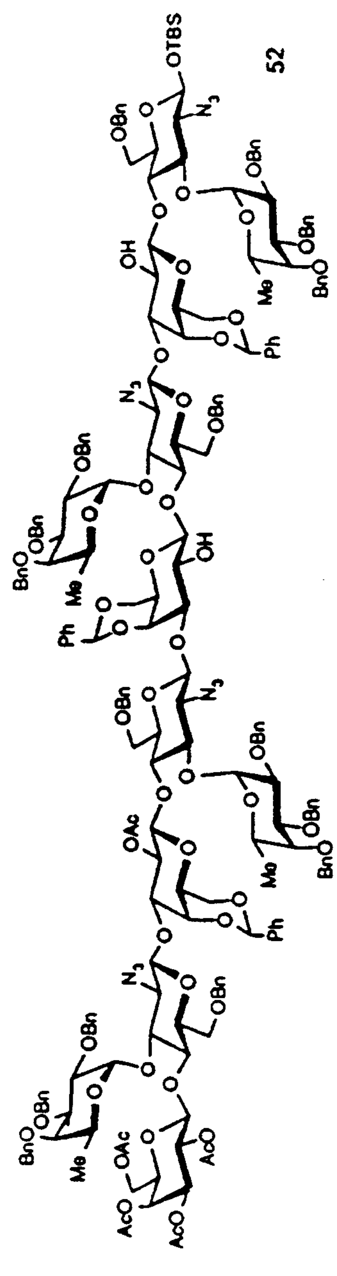

52

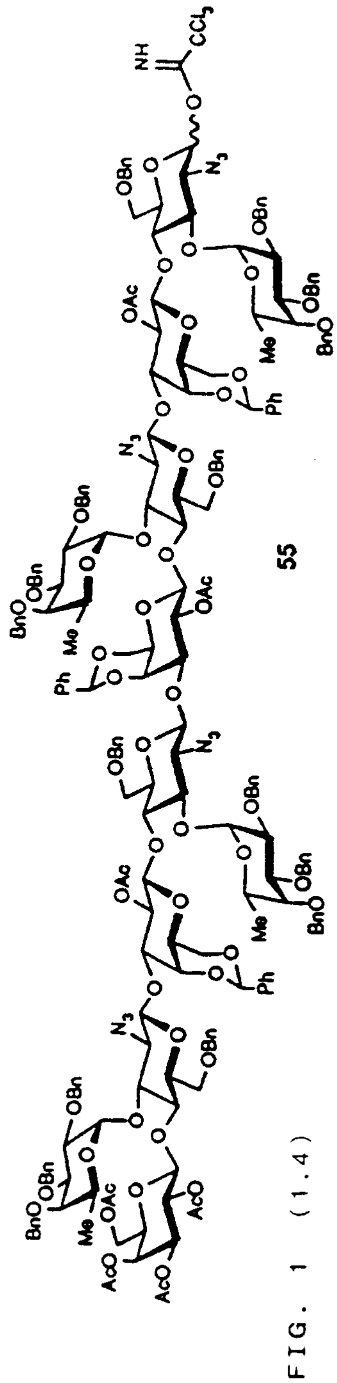

55

FIG. 1 (1.4)

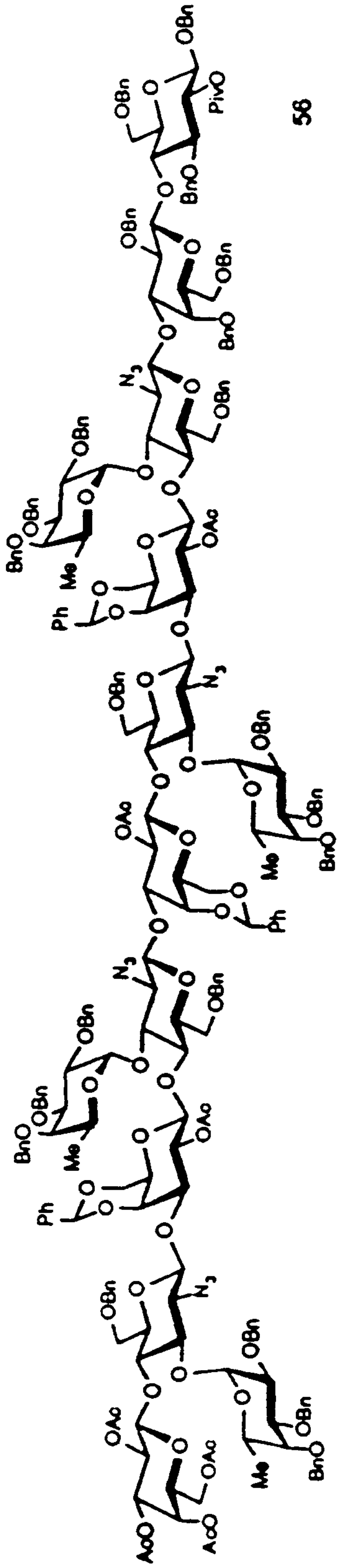

56

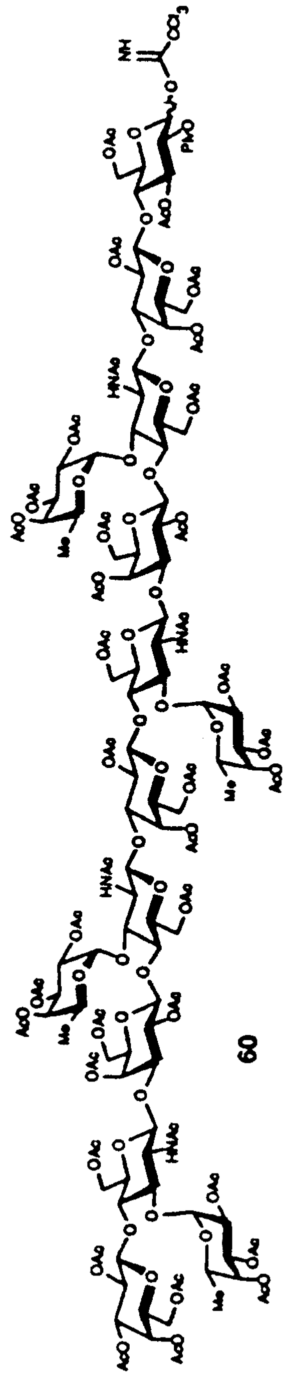

60

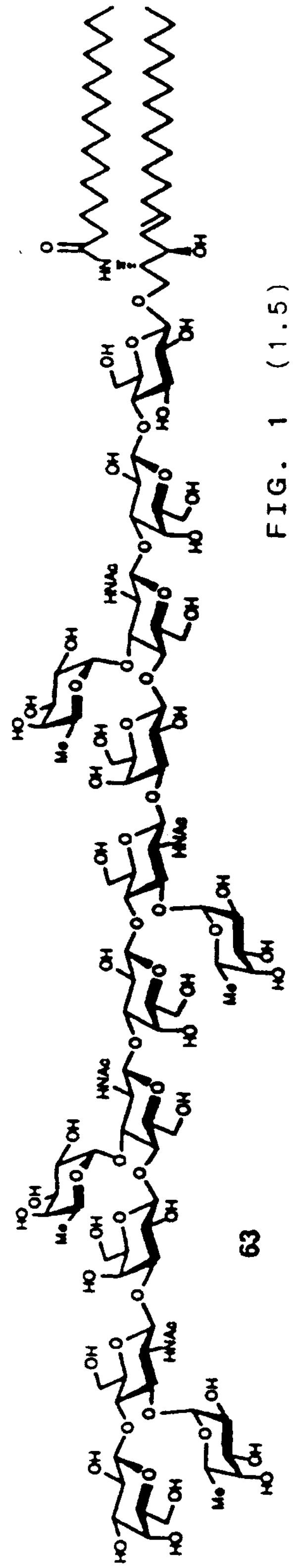

63

FIG. 1 (1.5)

Europäisches Patentamt

# EUROPÄISCHER RECHERCHENBERICHT

Nummer der Anmeldung

EP 93 10 9694

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | LIEBIGS ANN. CHEM. 1991, Seiten 425 - 433 R. BOMMER ET AL. 'Synthesis of the Octasaccharide Moiety of the Dimeric LeX Antigen' * das ganze Dokument * --- | 1-8 | C07H15/10 C07H13/04 C07H15/04 A61K31/70 |
| Y,D | J. AM. CHEM. SOC. Bd. 112, 1990, Seiten 3693 - 3695 K.C. NICOLAOU ET AL. 'Total Synthesis of the Tumor-Associated LeX Family of Glycosphingolipids' --- | 1-8 | |
| Y,D | SYNLETT 1990, Seiten 694 - 696 R.R. SCHMIDT ET AL. 'Nitriles as Solvents in Glycosylation Reactions: Highly Selective beta-Glycoside Synthesis' * das ganze Dokument * --- | 1-8 | |
| | | | RECHERCHIERTE SACHGEBIETE (Int. Cl.5) |
| P,X | TETRAHEDRON LETTERS Bd. 33, 1992, Seiten 5161 - 5164 A. TOEPFER ET AL. 'An Efficient Synthesis of the Lewis X (LeX) Antigen Family' * das ganze Dokument * ----- | 1-16 | C07H A61K |

Der vorliegende Recherchenbericht wurde für alle Patentansprüche erstellt

| Recherchenort | Abschlußdatum der Recherche | Prüfer |
|---|---|---|
| DEN HAAG | 14 OKTOBER 1993 | BRENNAN J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument